# EUROPEAN PATENT APPLICATION

(11) **EP 4 541 885 A1**
(43) Date of publication of application: **23.04.2025**
(21) Application number: 23306843.6
(22) Date of filing: 20.10.2023
(51) Int. Cl.: C12N 9/10, C12N 9/88, C12N 15/52, C12N 15/63, C12N 15/81, C12N 15/90, C12P 7/22

(54) **OPTIMIZED PHLOROGLUCINOL SYNTHASES**

(71) Applicant: COMPAGNIE GENERALE DES ETABLISSEMENTS MICHELIN, 63000 Clermont-Ferrand (FR)
(72) Inventor: The designation of the inventor has not yet been filed
(74) Representative: Regimbeau

(57) **Abstract**

The present invention relates to a mutant polypeptide comprising an amino acid sequence of a phloroglucinol synthase having at least 80 % of identity with the amino acid sequence of the *Tsukamurella pulmonis* phloroglucinol synthase as set forth in SEQ ID NO: 1, with one or more substitution(s) of one or more amino acid residue(s). The present invention also relates to a nucleic acid molecule encoding the mutant polypeptide, and also to a vector and a host cell comprising such nucleic acid molecule. The present invention also relates to methods for producing phloroglucinol.

## Description

### TECHNICAL FIELD OF THE INVENTION

The present invention relates to the fields of microbial biochemistry and more particularly to the field of the synthesis of phloroglucinol by microbial enzymes. The invention notably concerns optimized, mutated, phloroglucinol synthase, in particular bacterial optimized, mutated, phloroglucinol synthase, in particular from optimized, mutated, phloroglucinol synthase from actinomycete bacteria phloroglucinol synthase, especially from optimized, mutated, phloroglucinol synthase from phloroglucinol synthase of *Tsukamurella* bacteria, as well as methods and uses thereof to produce phloroglucinol.

### BACKGROUND ART

Phloroglucinol (Phlo) (1,3,5-benzenetriol according to chemical nomenclature) is an aromatic organic compound used in particular in the production of pharmaceutical products and explosives.

Phloroglucinol synthesis is catalysed by type III polyketide synthases known as phloroglucinol synthases. Phloroglucinol synthases carry out the condensation of three malonyl-CoA molecules so as to form a phloroglucinol molecule according to the following reaction scheme (Reaction I):

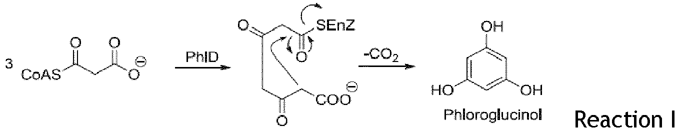

Numerous oligomers can subsequently be synthesized from phloroglucinol, such as phlorotannins. Phlorotannins include in particular fucols, phloretols and fucophloretols, which are phloroglucinol-derived products that make up the wall of brown algae. In addition, various protective activities of brown algae have also been attributed to phlorotannins.

Phloroglucinol synthesis has been described in Gram- *Pseudomonas fluorescens* bacteria (Achkar et al., 2005; Zha et al., 2006) and in the brown alga *Ectocarpus siliculosus* (Meslet-Cladière et al., 2013). In *Pseudomonas fluorescens,* the phloroglucinol synthase is encoded by the PHLD gene (Achkar et al., 2005; Zha et al., 2006). In *Ectocarpus siliculosus*, the phloroglucinol synthase is encoded by the PKS1 gene (Meslet-Cladière et al., 2013). PHLD phloroglucinol synthase activity has been demonstrated in *Escherichia coli* expressing a heterologous PHLD gene (Achkar et al., 2005). This activity has been confirmed *in vitro,* by means of small-scale enzymatic tests carried out with a recombinant PHLD expressed and purified from *Escherichia coli* cultures (Zha et al., 2006). PKS1 phloroglucinol synthase activity has been demonstrated *in vitro,* from recombinant PKS1 expressed and purified in *Escherichia coli* and from cell extracts of *E. siliculosus* (Meslet-Cladière et al., 2013, WO 2013/045510).

However, the PHLD and PKS1 enzymes exhibit low enzymatic activities. In addition, the possibility of synthesizing phloroglucinol *in vitro* on a large scale using these enzymes has not been proved to date. Finally, the phloroglucinol synthases used in these studies were produced by *E. coli* or *P. fluorescens* bacteria. The activity of these enzymes when they are produced by eukaryotes, such as yeasts or insect or mammalian cells, is thus unknown. Yet, the eukaryotic systems may be advantageous, in particular for large-scale productions. Other advantages of production in eukaryotes is their capacity to modify proteins, including enzymes, at the post-translational level.

Phloroglucinol synthases have been also identified in actinomycete bacteria (WO2019/002799) and in ochrophyte algae (WO 2019/002798). Among these, the phloroglucinol synthase of the actinomycete bacteria *Tsukamurella pulmonis* (Phld_Tpu, WT) was previously identified as a good candidate for phloroglucinol production in the yeast *Saccharomyces cerevisiae* (WO2019/002799).

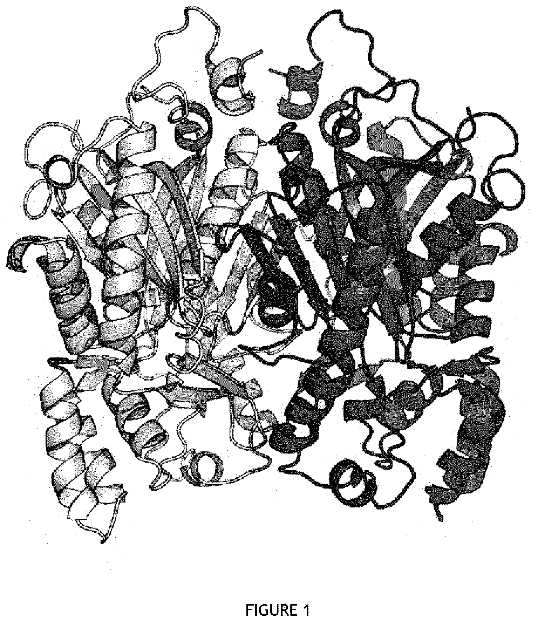

PhlD_Tpu amino acid sequence (as shown SEQ ID NO: 1) is 390 amino acids long (41 kDa) and is encoded by the gene SAMN04489765_2627 (access number for the protein: WP_068567598 NCBI database). It is assumed that the enzyme is biologically active in a homodimeric form, similarly to the other enzymes from the type III polyketide synthase (PKS) family to which Phld_Tpu belongs (**Figure 1**). This enzyme is directly involved in the phloroglucinol synthesis from the condensation of three molecules of malonyl-CoA (MLC).

While Phld_Tpu shows a satisfactory level of Phlo production in *S*. *cerevisiae,* there is still the need to identify new phloroglucinol synthases with optimised activity, which are suitable for large industrial-scale production, which can be produced by eukaryotic systems, and which have a high phloroglucinol synthesis enzymatic activity *in vitro* or *in vivo.*

However, the catalytic sites associated with phloroglucinol synthase activity have not been described, and no structural domain or motif, conserved between phloroglucinol synthases has been identified, making the design of optimized phloroglucinol synthases particularly difficult.

### SUMMARY OF THE INVENTION

In the context of the present invention, the Inventors have developed novel phloroglucinol synthases with optimised activity, that are particularly suitable for large industrial-scale production.

The Inventors designed an original and comprehensive mutagenesis strategy to identify mutant polypeptides from the phloroglucinol synthase of the actinomycete bacteria *Tsukamurella pulmonis* (Phld_Tpu, wild type (WT)), with improved properties, including increased phloroglucinol synthase activity.

In particular, state-of-the-art computational studies were used to predict Phld_Tpu three-dimensional structure in its functional homodimeric form and to uncover molecular determinants involved in substrate and product recognition. Using an innovative combination of computer-aided methods, several amino acid positions were predicted as targets for mutagenesis to improve catalytic activity and stability of the enzyme. Several libraries of mutants were then constructed and screened for improved Phlo production.

This strategy made of complementary approaches led to the identification of several simple mutants, as well as mutants with double or even multiple mutations, having a Phld activity (i.e. an activity of phloroglucinol production) significantly higher compared to WT Phld_Tpu. Indeed, the novel phloroglucinol synthase mutants allow to obtain concentrations of phloroglucinol up to 90% higher than those obtained with the wild type Phld, using the same conditions (i.e., the Inventors were able to obtain concentrations of phloroglucinol of up to 190% of concentrations of phloroglucinol obtained using the wild type, with the same experimental settings). The Inventors unexpectedly identified several key amino acid residues, allowing optimisation of the enzymatic activity.

The Inventors demonstrate here that these phloroglucinol synthase mutants are functional when they are produced in a prokaryotic system as well as an eukaryotic system and are suitable for large industrial-scale production.

The present invention thus relates to a mutant polypeptide comprising an amino acid sequence of a phloroglucinol synthase having at least 80% (preferably at least 85% more preferably at least 90 %) of identity with the amino acid sequence of the *Tsukamurella pulmonis* phloroglucinol synthase as set forth in SEQ ID NO: 1, with one or more substitution(s) of one or more amino acid residue(s) selected from the group consisting of:
a) any amino acid residue or combination of amino acid residues located from position 268 to position 271 of SEQ ID NO:1; or
   any amino acid residue or combination of amino acid residues located from a position equivalent to position 268 to a position equivalent to position 271 of SEQ ID NO: 1, after optimal global alignment with SEQ ID NO:1; or
b) an amino acid residue located at position 49 of SEQ ID NO:1; or
   an amino acid residue located at a position equivalent to position 49 of SEQ ID NO:1, after optimal global alignment with SEQ ID NO:1; or
c) an amino acid residue located at position 339 of SEQ ID NO:1; or
   an amino acid residue located at a position equivalent to position 339 of SEQ ID NO:1, after optimal global alignment with SEQ ID NO:1; or
d) amino acid residues located at positions 93 and 262 of SEQ ID NO:1; or
   amino acid residues located at positions equivalent to positions 93 and 262 of SEQ ID NO:1, after optimal global alignment with SEQ ID NO:1; or
e) amino acid residues located at positions 129 and 176 of SEQ ID NO:1; or
   amino acid residues located at positions equivalent to positions 129 and 176 of SEQ ID NO:1, after optimal global alignment with SEQ ID NO:1; and
f) any combination thereof.

The present invention also relates to nucleic acid molecules encoding these mutant polypeptides, in particular encoding mutant phloroglucinol synthases obtained from wild type phloroglucinol synthase of *T. pulmonis* (Phld_Tpu, SEQ ID NO:1). The invention concerns more specifically a nucleic acid molecule comprising a nucleic acid sequence encoding the mutant polypeptide of the invention, preferably wherein:
a) the nucleic acid molecule is isolated; or
b) the nucleic acid molecule further comprises a promoter controlling expression of the nucleic acid sequence; or
c) the isolated nucleic acid molecule further comprises a transcription terminator controlling expression of the nucleic acid sequence; or
d) the nucleic acid sequence is further optimized for expression in a host cell, in particular a yeast or a bacterium; or
e) any combination of a) to d).

The invention also relates to vectors comprising at least one nucleic acid molecule encoding such a mutant polypeptide.

The invention also relates to host cells comprising at least one nucleic acid molecule or at least one vector according to the invention.

The invention also relates to methods for producing a functional phloroglucinol synthase.

The invention also relates to methods and uses for producing phloroglucinol.

### DETAILED DESCRIPTION OF THE INVENTION

In the context of the present invention, the inventors have developed novel phloroglucinol synthases (Phld) with optimised activity, that are particularly suitable for large industrial-scale production.

The Inventors designed an original and comprehensive mutagenesis strategy to identify mutant polypeptides from the phloroglucinol synthase of the actinomycete bacteria *Tsukamurella pulmonis* (Phld_Tpu, wild type (WT)), with improved properties, including increased phloroglucinol synthase activity.

In particular, state-of-the-art computational studies were carried out to predict Phld_Tpu three-dimensional structure in its functional homodimeric form and to uncover molecular determinants involved in substrate and product recognition. Using these original computer-aided strategies, several amino acid positions were predicted as targets for mutagenesis to improve catalytic activity and stability of the enzyme. Several libraries of mutants were then constructed and screened for improved Phlo production.

This combination of complementary strategies led to the identification of several simple mutants, as well as mutants with double or even multiple mutations, having a Phlo production significantly higher compared to WT Phld_Tpu. Indeed, the novel phloroglucinol synthase mutants allow to obtain concentrations of phloroglucinol up to 90% higher than those obtained with the wild type Phld, using the same conditions (i.e., the Inventors were able to obtain concentrations of phloroglucinol of up to 190% of concentrations of phloroglucinol obtained using the wild type, with the same experimental settings). The Inventors unexpectedly identified several key amino acid residues, allowing optimisation of the enzymatic activity. The Inventors demonstrate here that these mutant phloroglucinol synthases are functional when they are produced in a prokaryotic system as well as an eukaryotic system and are suitable for large industrial-scale production.

### Definitions

Unless specifically defined herein, all technical and scientific terms used herein have the same meaning as commonly understood by a skilled artisan in chemistry, biochemistry, microbiology, cellular biology, molecular biology, and medical sciences.

As used herein throughout the entire text, the terms "**a**" and "**an**" are used in the sense that they mean "at least one", "at least a first", "one or more" or "one or a plurality" of the referenced compounds or steps, unless the context dictates otherwise.

The term "**and/or**" wherever used herein includes the meaning of "and", "or" and "all or any other combination of the elements connected by said term".

The term "a**b**out" or "approximately" as used herein means within 10%, preferably within 8%, and more preferably within 5%, and more preferably within 3%, and more preferably within 1% of a given value or range.

As used herein, when used to define products, compositions, cells, uses and methods, the term "**comprising**" (and any form of comprising, such as "comprise" and "comprises"), "**having**" (and any form of having, such as "have" and "has"), "**including**" (and any form of including, such as "includes" and "include") or "**containing**" (and any form of containing, such as "contains" and "contain") are open-ended and do not exclude additional, unrecited elements or method steps. Thus, a polypeptide "comprises" an amino acid sequence when the amino acid sequence might be part of the final (and/or whole) amino acid sequence of the polypeptide. Such a polypeptide can have up to several hundred additional amino acid residues (e.g., corresponding to linker(s), tag(s) and any other moiety as described herein). "**Consisting of**" means excluding any other components or steps "**Consisting essentially of**" means excluding other components or steps of any essential significance (however, other minor/insignificant components or steps are not excluded). In the present disclosure, the terms "comprising", "consisting of" and "consisting essentially of" may be replaced with each other, if required.

The terms **"type III polyketide synthase"** herein refer to a multifunctional enzyme or an enzymatic complex producing polyketides and which does not use an acyl carrier protein (or ACP) domain.

The term **"polyketide"** is intended to mean a large family of secondary metabolites in many eukaryotes and prokaryotes, including bacteria, fungi, algae, plants and animals, which originate from the iterative condensation of acetyl or malonyl subunits by polyketide-synthase enzymes. Polyketides also serve as starting materials for the production of a wide range of natural and semi-synthetic products.

The term **"phloroglucinol"** (abbreviated as "Phlo") herein means an aromatic organic compound **benzene-1,3,5-triol** having the following chemical formula (formula I) and a CAS number 108-73-6:

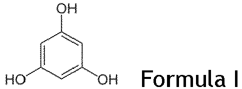

The term **"phloroglucinol synthase"** (abbreviated as PHLD or PhlD) is intended to mean a multifunctional enzyme or an enzymatic complex which belongs to the family of **type III polyketide synthases** and which catalyses phloroglucinol synthesis. A phloroglucinol synthase catalyses the condensation of three malonyl-CoA molecules so as to form a phloroglucinol molecule.

As used herein, the term "**enzyme**" refers to a polypeptide with catalytic properties. An enzyme acts by reducing the activation energy of a chemical reaction, thereby increasing the speed of the reaction. The enzyme is not modified during the reaction. The initial molecules are the enzyme's "substrates", and the molecules formed from these substrates are the "reaction products". Enzymes are highly specific. What's more, enzymes are reusable. Enzymes are generally globular proteins that act alone or in complexes of several enzymes or subunits. Like all proteins, enzymes are made up of one or more polypeptide chains folded to form a three-dimensional structure corresponding to their native state. The size of enzymes can vary from around 50 to over 2000 residues. Only a very small part of the enzyme - usually between two and four residues, sometimes more - is directly involved in catalysis, and these constitute the so-called catalytic site. The catalytic site is usually located close to one or more binding sites, where the substrate(s) is (are) bound and oriented to catalyze the chemical reaction. The catalytic site and the binding site(s) form the enzyme's active site.

The term **"enzymatic activity"** or **"catalytic activity"** or else **"activity"** of an enzyme refers to the amount of product formed or substrate consumed (in mole, micromole or gram/l) per unit time (sec, min or days) in a given environment. The expression **"degree of conversion of the substrate into a product by the enzyme"** is intended to mean here the ratio between the amount of final product obtained relative to the initial amount of substrate for a defined amount of enzyme. For example, for the purposes of the invention, an enzymatic activity can be expressed as an amount of phloroglucinol produced in a given volume per unit of time (in g/l/time).

The term **"bacterium"** herein means a microscopic and prokaryotic organism present in all media.

The term **"Gram+ bacterium"** or **"Gram-positive bacterium"** herein means a bacterium which is positive upon Gram staining (that is to say which retains the gentian violet, also known as crystal violet, and remains mauve-violet-coloured or blue-dark purple-coloured).

The term **"actinomycete bacterium"** or **"Gram+ actinomycete bacterium"** or **"Gram-positive actinomycete bacterium"** herein means a bacterium belonging to the order *Actinomycetales* in the classification of actinobacteria, which is positive with respect to Gram staining. Actinomycetes are bacteria, the growth of which give rise to colonies consisting of hyphae, that is to say filaments which radiate, by centrifugal growth, all around the microorganism which gave rise to them.

The term ***"Tsukamurella sp."*** herein means a rod-shaped, necessarily aerobic, non-sporulating actinomycete bacterium of the *Tsukamurella* genus. The *Tsukamurella* genus comprises in particular the species *Tsukamurella paurometabola* (also referred to as Tp hereinafter), *Tsukamurella tyrosinosolvens* (also referred to as Tt hereinafter), *Tsukamurella pseudospumae* (also referred to as Tps hereinafter), *Tsukamurella pulmonis* (also referred to as Tpu hereinafter) and *Tsukamurella sp.* 1534 (also referred to as Tsp hereinafter).

The term ***"Nocardia sp."*** herein means a filamentous actinomycete bacterium of the *Nocardia* genus. The *Nocardia* genus comprises in particular the species *Nocardia farcinica* (also referred to as Nf hereinafter).

The term ***"Mycobacterium sp."*** herein means a non-sporulating, aerobic actinomycete bacterium, in the shape of bacilli, of the *Mycobacterium* genus. The *Mycobacterium* genus comprises in particular the species *Mycobacterium marinum, Mycobacterium kansasii* and *Mycobacterium tuberculosis* (also respectively referred to as Mma, Mk and Mt hereinafter).

The term ***"Gordonia sp."*** herein means an actinomycete bacterium of the *Gordonia* genus. The *Gordonia* genus comprises in particular the species *Gordonia hydrophobica* (also referred to as Gh hereinafter).

The term ***"Pseudomonas sp."*** herein means a Gram-negative (Gram-) bacterium, which does not form spores (or non-sporulating), which is in the form of a bacillus and which is necessarily aerobic, of the *Pseudomonas* genus. The *Pseudomonas* genus comprises in particular the species *Pseudomonas fluorescens* (also known as Pf hereinafter).

The term ***"Ectocarpus sp."*** herein means an alga of the ***Ectocarpus*** genus, of the class of brown algae, belonging to the family ***Ectocarpaceae.*** The ***Ectocarpus*** genus comprises in particular the species ***Ectocarpus siliculosus.***

The term **"PHLD.Pf"** herein means, without distinction, the gene encoding the PHLD phloroglucinol synthase of *P. fluorescens,* or the polypeptide encoded by this gene.

The term **"PKS1.Es"** or **"PHLD.Es"** is intended to mean, without distinction, the gene encoding the PKS1 phloroglucinol synthase of *E. siliculosus,* or the polypeptide encoded by this gene.

The term **"PhlD_Tpu"** or **"PHLD_Tpu"** denotes here the wild type, unmutated phloroglucinol synthase of *Tsukamurella pulmonis,* having the amino acid sequence shown in SEQ ID NO:1.

The terms **"obtained from", "originating from", "originate from",** or **"of XXX origin"** (wherein XXX is any source) are used to identify the original source of a component (e.g., polypeptide, nucleic acid molecule) but are not meant to limit the method by which the component is made which can be, for example, by chemical synthesis, mutations, and/or recombinant means (including replacement of codon encoding amino acid residues).

The terms **"peptide", "polypeptide"** and **"protein"** are used interchangeably herein and refer to any polymer of covalently linked amino acids, regardless of length or post-translational modification. No limitation is placed on the maximum number of amino acids comprised in a polypeptide. As a general indication, the terms refer to both short polymers (typically designated in the art as peptide, or protein fragment) and longer polymers (typically designated in the art as polypeptide or protein). As a general indication and without being bound therein, if the amino acid polymer contains more than 50 amino acid residues, it is preferably referred to as a polypeptide or a protein, whereas if the polymer consists of 50 or fewer amino acids, it is preferably referred to as a "peptide".

A polypeptide may be any translational product of a polynucleotide regardless of size. Alternatively, a polypeptide may be any product of a chemical synthesis reaction.

The polymer can be linear, branched or cyclic, preferably linear. The polymer may comprise naturally occurring amino acids and/or amino acid analogues and it may be interrupted by non-amino acids.

Amino acids in a polypeptide are typically covalently linked by peptide bonds. Preferably, all the chemical bonds in a polypeptide are peptide bonds. In some instances, the polypeptide may comprise one or more chemical bonds which are not peptide bonds. The term "polypeptide" encompasses native polypeptides, as well as non-native polypeptides, including derivatives, mutated polypeptides, engineered polypeptides, fusion polypeptides, among others. The term "polypeptide" also encompasses polypeptide fragments and polypeptide multimers (e.g. dimers), including homo- and hetero-multimers. Polypeptides usable herein can be further modified by chemical or enzymatic modification. Such a chemically and/or enzymatically modified polypeptide comprises chemical groups other than the chemical groups of the 20 naturally occurring amino acids. Examples of such chemical or enzymatic modifications include post-translational modifications, addition of a label/tag, etc. Chemical or enzymatic modifications of a polypeptide may alter one or more property(ies) of the polypeptide. For example, some modifications may alter stability, biological half-life, solubility (such as water solubility), activity, etc.

The reading and writing senses of an amino acid sequence of a polypeptide as used herein are the conventional reading and writing senses. The reading and writing convention for amino acid sequences of a polypeptide places the amino terminus on the left, with the sequence then being written and read from the amino terminus (N-terminus) to the carboxyl terminus (C-terminus), from left to right.

The terms **"amino acids", "residues"** and **"amino acid residues"** are used interchangeably and encompass natural amino acids as well as amino acid analogs obtained after the withdrawal of one molecule of water (e.g., non-natural, synthetic and modified amino acids, including D or L optical isomers). The terms **"amino acids"** are used interchangeably and encompass intact natural amino acids as well as amino acid analogs (e.g., non-natural, synthetic and modified amino acids, including D or L optical isomers).

The terms **"peptide tag"** or **"tag peptide"** or **"peptide label"** or **"label peptide"** refer to a short amino acid sequence (in particular 2 to 25 amino acids) fused to another polypeptide and used to detect the presence of this polypeptide, or to facilitate its purification or solubilization. Examples of such label peptides are described in Kimple et al [Kimple ME, et al. 2013] and presented in **Table** 1 below.

**Table 1 : Examples of peptide tags**

| Name | Structure/sequence | Sequence identifier |
|---|---|---|
| polyhistidine | generally 2 to 10 consecutive histidine residues, more often 5 or 6 consecutive histidine residues | / |
| polyarginine | generally 2 to 10 consecutive arginine residues, more often 5 or 6 consecutive arginine residues | / |
| polyaspartate | generally 2 to 16 consecutive aspartate residues, more often 5 or 6 consecutive aspartate residues | / |
| polycysteine | generally 2 to 10 consecutive cysteine residues, more often 4 or 5 consecutive cysteine residues | / |
| polyphenylalanine | generally 4 to 10 consecutive phenylalanine residues, more often 11 consecutive phenylalanine residues | / |
| FLAG peptide | DYKDDDK | SEQ ID NO :34 |
| Strep-tag | WSHPQFEK or AWAHPQPGG | SEQ ID NO: 35 or SEQ ID NO: 36 |
| B-tag | QYPALT | SEQ ID NO : 37 |
| EE-tag | EYMPME or EFMPME | SEQ ID NO : 38 or SEQ ID NO : 39 |
| T7-tag | MASMTGGQQMG | SEQ ID NO : 40 |
| V5-tag | GKPIPNPLLGLDST | SEQ ID NO : 41 |
| E2 | SSTSSDFRDR | SEQ ID NO : 42 |
| HAT | KDHLIHNVHKEFHAHAHNK | SEQ ID NO : 43 |
| AU1 | DTYRYI | SEQ ID NO : 44 |
| AU5 | TDFYLK | SEQ ID NO : 45 |

As used herein, **"post-translational modification"** refers to a chemical or enzymatic modification occurring naturally or not on a protein or a protein fragment, after or concomitantly to translation (e.g., biological or biochemical protein synthesis, e.g., using cellular machinery), or after or concomitantly to protein synthesis (e.g., artificial and/or chemical synthesis). This means that at least one of the naturally occurring amino acid of the protein or protein fragment is modified by the addition of at least one chemical group and/or the modification (including, but not limited to, the removal) of at least one chemical group of the naturally occurring amino acid. Examples of such chemical or enzymatic modifications include without limitation glycosylation, phosphorylation, acylation, carboxylation, acetylation, biotinylation, hydroxylation, lipoylation, amidation, ubiquitination, sumoylation, deamination, etc. By **"post-translationally modified protein"** it is herein referred to a protein having at least one (i.e., one or more) post-translational modification. By **"post-translationally modified protein fragment"** it is herein referred to a protein fragment having at least one (i.e., one or more) post-translational modification.

The terms **"polynucleotide", "nucleic acid molecule",** and **"nucleic acid"** are used interchangeably herein and are understood as a polymeric or oligomeric macromolecule made from nucleotide monomers (preferably from at least 5 nucleotide monomers, also called nucleotide residues). Nucleotide monomers are composed of a nucleobase, a five-carbon sugar (such as but not limited to ribose or 2'-deoxyribose), and one to three phosphate groups. Nucleotide monomers can be chemically and/or enzymatically modified. Typically, a polynucleotide is formed through phosphodiester bonds or phosphorothioate bonds between the individual nucleotide monomers. Nucleic acid molecules include, but are not limited to, ribonucleic acid (RNA), deoxyribonucleic acid (DNA), and mixtures thereof such as e.g., RNA-DNA hybrids (mixed polyribo-polydeoxyribonucleotides). These terms encompass single or double-stranded, linear or circular, natural or synthetic, unmodified or modified versions thereof (e.g., genetically modified polynucleotides; optimized polynucleotides), sense or antisense polynucleotides, chimeric mixture (e.g., RNA-DNA hybrids). Moreover, a polynucleotide may comprise non-naturally occurring nucleotides and may be interrupted by non-nucleotide components. Exemplary DNA nucleic acids include without limitations, complementary DNA (cDNA), genomic DNA, plasmid DNA, DNA vector, viral DNA (e.g., viral genomes, viral vectors), oligonucleotides, probes, primers, satellite DNA, microsatellite DNA, coding DNA, non-coding DNA, antisense DNA, and any mixture thereof. Exemplary RNA nucleic acids include, without limitations, messenger RNA (mRNA), precursor messenger RNA (pre-mRNA), small interfering RNA (siRNA), short hairpin RNA (shRNA), microRNA (miRNA), RNA vector, viral RNA, guide RNA (gRNA), antisense RNA, coding RNA, non-coding RNA, antisense RNA, satellite RNA, small cytoplasmic RNA, small nuclear RNA, etc. Polynucleotides described herein may be synthesized by standard methods known in the art, e.g., by use of an automated DNA synthesizer (such as those that are commercially available from Biosearch, Applied Biosystems, etc.) or obtained from a naturally occurring source (e.g., a genome, cDNA, etc.) or an artificial source (such as a commercially available library, a plasmid, etc.) using molecular biology techniques well known in the art (e.g., cloning, PCR, etc.). The nucleic acids, can e.g., be synthesized chemically, e.g., in accordance with the phosphotriester method (see, for example, Uhlmann, E. & Peyman, A. (1990) Chemical Reviews, 90, 543-584).

The term **"vector"** is intended to mean a carrier, preferably a nucleic acid molecule or a viral particle, which contains the elements required to enable one or more nucleic acid molecule(s) to be administered into, propagated in and/or expressed in a host cell or an organism.

From a functional point of view, this term encompasses maintenance vectors (cloning vectors), vectors for expression in various host cells or organisms (expression vectors), extrachromosomal vectors (for example multicopy plasmids) or integrating vectors (for example designed to integrate into the genome of a host cell and to produce additional copies of the nucleic acid molecule that it contains when the host cell replicates). This term also encompasses shuttle vectors (for example, which function both in prokaryotic hosts and/or eukaryotic hosts) and transfer vectors (for example for the transfer of nucleic acid molecule(s) into the genome of a host cell).

From a structural point of view, the vectors according to the invention may be natural, synthetic or artificial genetic sources, or a combination of natural and artificial genetic elements.

Thus, in the context of the invention, the term "vector" should be understood broadly while including plasmid vectors (or plasmids) and viral vectors.

A **"plasmid"** as used here denotes a replicable DNA construct. Usually, plasmid vectors contain selectable marker genes which allow the host cells carrying the plasmid to be identified and/or selected positively or negatively in the presence of the compound corresponding to the selectable marker. A variety of positive and negative selectable marker genes are known in the art. For illustrative purpose, an antibiotic resistance gene can be used as a positive selectable marker gene for selecting a host cell in the presence of the corresponding antibiotic.

The term **"viral vector"** as used here refers to a nucleic acid vector which comprises at least one element of a viral genome and which can be packaged in a viral particle, or a viral particle. The viral vectors may be replication-competent or selective (for example, designed to replicate better or selectively in specific host cells), or may be genetically deactivated so as to be defective or deficient for replication.

As used herein, the term **"host cell"** should be understood broadly without any limitation concerning particular organization in tissue, organ, or isolated cells. The term **"host cell"** is intended to mean a cell containing a polypeptide according to the invention, a nucleic acid molecule according to the invention, a vector according to the invention, or any mixture of these. Advantageously, the host cell is capable of expressing a polypeptide with phloroglucinol synthase activity and/or of producing the vector of the invention. Advantageously again, the host cell is capable of synthesizing phloroglucinol.

The host cell can be a producer cell, i.e., a cell capable of expressing the nucleic acid molecule(s) (including gene(s)) encoded by the vector according to the invention and/or of producing the vector of the invention (i.e., a cell expressing the nucleic acid molecule(s) (including gene(s)) encoded by the vector according to the invention and/or producing the vector of the invention). This term also includes cells that express the polypeptide of the invention, and cells that can be or have been the recipient of the nucleic acid molecule encoding the polypeptide of the invention (or the vector comprising said nucleic acid molecule), as well as progeny of such cells. The term "host cell" more broadly comprises cells which contain or have contained the nucleic acid molecule according to the invention, as well as descendants of such cells.

The host cell can be made up of a single type of cells or a group of different types of cells. The host cell can also be a hybrid cell, i.e., resulting from the fusion of at least two cells of different types. The host cell can be isolated, for example, or organized in tissue, organ or even a within complete organism. In the case where the host cell is a within complete organism, said organism is not human.

The host cell can belong to cultured cell lines, primary cells, stem cells, proliferative cells, or dividing cells. The term "host cells" comprises prokaryotic cells, lower eukaryotic cells such as yeast cells, and other eukaryotic cells such as archaebacteria cells, fungus cells, insect cells, plant cells, algae cells, microalgae cells, parasite cells, animal cells and mammalian cells (for example, human or non-human, preferably non-human). The host cell can be a differentiated cell, a pluripotent cell, a totipotent cell, a stem cell, an induced pluripotent stem cell (iPSC), an induced totipotent stem cell, or even an embryonic cell or embryonic stem cell. In the case of an embryonic cell or embryonic stem cell, the cell is a non-human cell.

It is thus clear that a "host cell" according to the present invention is a recombinant host cell, i.e. a cell housing an exogenous genetic material. Thus, a host cell is not a cell that exists naturally but is a molecular biology tool obtained by genetic manipulation techniques.

The term **"isolated molecule"** is intended to mean a molecule, in particular a protein, a polypeptide, a peptide, a nucleic acid molecule, a plasmid vector, a viral vector or a host cell, which is extracted from its natural environment (that is to say separated from at least one other component with which it is naturally associated).

As used herein, **"identity"** or **"sequence identity"** means an exact sequence match between two polypeptides or amino acids, or between two nucleic acid molecules or oligonucleotides. The **"percent identities"** referred to in the context of the disclosure of the present invention are determined after optimal alignment of the sequences to be compared, which optimal global alignment may therefore comprise one or more insertions, deletions, truncations and/or substitutions. The alignment is "global", meaning that it includes the sequences to be compared taken in their entirety over their entire length. The alignment is "optimal", meaning that the number of insertions, deletions, truncations and/or substitutions is made as low as possible. The optimal global alignment may be performed and the percent identity may be calculated using any sequence analysis method well-known to the person skilled in the art. In addition to manual comparison, it is possible to determine global alignment using the algorithm of Needleman and Wunsch (A general method applicable to the search for similarities in the amino acid sequence of two proteins, J. Mol. Biol., 1970, Mar;48(3):443-453). The optimal global alignment may be performed and the percent identity may be calculated using any software well-known to a person skilled in the art, such as the Emboss Needle software. The Emboss Needle software, for example, is available on the ebi.ac.uk world wide website under the name "Align". This software reads two input sequences and writes their optimal global sequence alignment to file. It uses the Needleman-Wunsch alignment algorithm to find the optimum alignment (including gaps) of two sequences along their entire length. The algorithm uses a dynamic programming method to ensure the alignment is optimum, by exploring all possible alignments and choosing the best. A scoring matrix is read that contains values for every possible residue or nucleotide match. Emboss Needle software finds the alignment with the maximum possible score where the score of an alignment is equal to the sum of the matches taken from the scoring matrix, minus penalties arising from opening and extending gaps in the aligned sequences. The substitution matrix and gap opening and extension penalties are user-specified. In the context of the invention, in order to obtain an optimal global alignment, the Emboss Needle software may be used with default parameters. For nucleotide sequences, the parameters used may notably be the following: "Gap open" equal to 10.0, "Gap extend" equal to 0.5, and the EDNAFULL matrix (NCBI EMBOSS Version NUC4.4). For amino acid sequences, the parameters used may notably be the following: "Gap open" equal to 10.0, "Gap extend" equal to 0.5, and the BLOSUM62 matrix; or the parameters used may be : the "Gap open" parameter equal to 10.0, the "Gap extend" parameter equal to 0.5, the "End gap penalty" parameter to "false", the "End gap open" parameter to 10.0, and a "Blosum 62" matrix. When entering two (or more) amino acid sequences or two (or more) nucleotide sequences, the Emboss Needle software returns an optimal global alignment, as well as several values characterizing the alignment:
- "Identity" is the percentage of identity, i.e. the percentage of identical matches between the two sequences over the reported aligned region (including any gaps in the length),
- "Similarity" is the percentage of similarity, i.e. the percentage taking into account both the identical matches between the two sequences over the reported aligned region (including any gaps in the length), and conservative substitutions.
- "Score" is the total score of the alignment, corresponding to the best score obtained by the software for all tested global alignments (i.e. over the entire length of both sequences).

This score may be referred to as a "similarity score", since the higher is the value of the score between the two (or more) sequences to align, the higher is the similarity between these two (or more) sequences, taking into account not only aligned identical amino acids, but also conservative substitutions.

This score may be calculated using any software well-known to a person skilled in the art, such as the Emboss Needle software, and may also be calculated manually.

Preferably, the percent identity as defined in the context of the present invention is determined via the global alignment of sequences compared over their entire length. For illustrative purposes, "at least 80% identity" herein means 80%, 81%, 82%, 83%, 84%, 85%, 86%, 87%, 88%, 89%, 90%, 91%, 92%, 93%, 94%, 95%, 96%, 97%, 98%, 99% or 100% identity.

An **"identical match"** between two (or more) residues (such as amino acid or nucleic acid residues) herein means that the two residues are identical.

A polypeptide or nucleic acid molecule **"mutant"** is herein defined as a polypeptide or nucleic acid molecule comprising one or more mutations relative to a reference amino acid or nucleic acid sequence. Mutations can include substitutions (replacement of one amino acid or nucleic acid by another amino acid or nucleic acid), deletions (deletion of one or more amino acid(s) or nucleic acid(s) at one of the ends or within the reference sequence) and/or insertions (addition of one or more amino acid(s) or nucleic acid(s) at one of the ends or within the reference sequence).

A mutant of a reference polypeptide preferably retains the activity of the reference polypeptide. A reference polypeptide may be for example a wild type polypeptide. In the case of an enzyme, a mutant preferably retains the enzymatic activity of the reference enzyme.

By **"silent mutation"** or **"silent substitution",** it is herein referred to the replacement of a nucleotide residue (also called "original nucleotide") in a protein-coding region of a nucleic acid molecule, by a different nucleotide residue, which does not affect the amino acid sequence encoded by the nucleic acid molecule. A silent mutation leads to a change of one of the letters in the triplet code that represents a codon, but despite the single base change, the amino acid that is coded for remains unchanged. This is permitted by the degeneracy of the genetic code. **Table 2** below shows an example of the standard genetic code, showing the codons encoding each of the standard amino acid residues.

**Table 2 : Standard genetic code**

| **1st base** | **2nd base** | | | | | | | | **3rd base** |
|---|---|---|---|---|---|---|---|---|---|
| | **T** | | **C** | | **A** | | **G** | | |
| **T** | TT T | (Phe/F) Phenylal anine | TC T | (Ser/S) Serine | TA | (Tyr/Y) Tyrosine | TG T | (Cys/C) Cysteine | **T** |
| | TT C | | TC C | | TA C | | TG C | | **C** |
| | TT A | (Leu/L) Leucine | TC A | | TAA | Stop (*Ochre*) | TGA | Stop (*Opal*)^{[} | **A** |
| | TT G | | TC G | | TA G | Stop (*Amber*) | TG G | (Trp/W) Tryptophan | **G** |
| **C** | CT T | | CC T | (Pro/P) Proline | CA T | (His/H) Histidine | CG T | (Arg/R) Arginine | **T** |
| | CT C | | CC C | | CA C | | CG C | | **C** |
| | CT A | | CC A | | CA A | (Gln/Q) Gluta mine | CG A | | **A** |
| | CT G | | CC G | | CA G | | CG G | | **G** |
| **A** | AT T | (Ile/I) Isoleucine | AC T | (Thr/T) Threo nine | AA T | (Asn/N) Aspar agine | AG T | (Ser/S) Serine | **T** |
| | AT C | | AC C | | AA C | | AG C | | **C** |
| | AT A | | AC A | | AA A | | AG A | (Arg/R) Arginine | **A** |
| | AT G | (Met/M) Methion ine | AC G | | AA G | | AG G | | **G** |
| **G** | GT T | | GC T | (Ala/A) Alanine | GA T | (Asp/D) Aspart ic acid | GG T | (Gly/G) Glycin e | **T** |
| | GT C | | GC C | | GA C | | GG C | | **C** |
| | GT A | | GC A | | GA A | (Glu/E) Gluta mic acid | GG A | | **A** |
| | GT G | | GC G | | GA G | | GG G | | **G** |

**"Conservative substitution"** or "conservative mutation" herein refers to the replacement of an amino acid residue (also called "original amino acid") in a polypeptide (e.g., a protein) by a different amino acid residue with similar physicochemical properties (molar mass, electrical charge, functional groups, hydrophobicity, etc.). **Table 3** below shows examples of equivalent amino acids that can be used in conservative substitutions.

**Table 3: Examples of amino acids with equivalent physicochemical properties**

| **Original amino acid** | **Equivalent amino acid** |
|---|---|
| Ala (A) | Val, Gly, Pro |
| Arg (R) | Lys, His |
| Asn (N) | Gln |
| Asp (D) | Glu |
| Cys (C) | Ser |
| Gln (Q) | Asn |
| Glu (G) | Asp |
| Gly (G) | Ala |
| His (H) | Arg, Lys |
| Ile (I) | Leu, Met, Val |
| Leu (L) | Ile, Val, Met |
| Lys (K) | Arg, His |
| Met (M) | Leu, Val, Ile |
| Phe (F) | Tyr, Trp |
| Pro (P) | Ala |
| Ser (S) | Thr, Cys |
| Thr (T) | Ser |
| Trp (W) | Tyr, Phe |
| Tyr (Y) | Phe, Trp |
| Val (V) | Leu, Ala, Ile |

**"Functional mutant/derivative of a polypeptide"** means any polypeptide, peptide, or protein fragment derived from a polypeptide, peptide, or protein fragment, having at least one of the original functions of the polypeptide, peptide, or protein from which it is derived. Preferably, the functional mutant/derivative performs said function with an efficiency equal to at least 30% of that of said peptide or protein or molecule, preferably at least 40%, preferably at least 45%, preferably at least 50%, preferably at least 55%, preferably at least 60%, preferably at least 65%, preferably at least 70%, preferably at least 75%, preferably at least 80%, preferably at least 85%, preferably at least 90%, preferably at least 91%, preferably at least 92%, preferably at least 93%, preferably at least 94%, preferably at least 95%, preferably at least 96%, preferably at least 97%, preferably at least 98%, preferably at least 99%, preferably at least 100% of the efficacy of said polypeptide, peptide, or protein. Examples of functional mutant/derivative of a polypeptide include e.g., mutated protein or polypeptide (including protein or polypeptide mutated by substitution, deletion, insertion, fusion, or any combination thereof, etc.), protein domains (such as protein enzymatic domains), protein epitopes, etc. Substitution comprises replacement of at least one amino acid residue by at least one distinct amino acid residue, preferably by one or more foreign protein, by one or more foreign protein fragment, or by one or more foreign polypeptide (i.e. wherein the protein, protein fragment, or polypeptide originates from another species). Insertion comprises insertion (addition) of at least one amino acid residue, preferably insertion of one or more foreign protein(s), of one or more foreign protein fragment, or of one or more foreign polypeptide (i.e. wherein the protein, protein fragment, or polypeptide originates from another species). Fusion comprises covalently bonding at least one amino acid residue, preferably covalently bonding one or more foreign protein(s), one or more foreign protein fragment(s), or one or more foreign polypeptide(s) (i.e. wherein the protein, protein fragment, or polypeptide originates from another species), using a linker sequence or not.

Substitution(s), insertion(s), deletion(s), and fusion(s) can be generated by a number of ways known to those skilled in the art, such as site-directed mutagenesis, PCR mutagenesis, DNA shuffling, chemical synthetic techniques (e.g., resulting in a synthetic nucleic acid molecule encoding the engineered protein), and/or using restriction sites, etc.

Functional mutants/derivatives usable herein can be further modified by chemical or enzymatic modification (e.g., post-translational modification(s)). A chemically/enzymatically modified polypeptide, peptide, or protein fragment comprise other chemical groups than those of the 20 naturally occurring amino acids (e.g., can comprise post-translational modification(s), and/or non-naturally occurring amino acids).

In the detailed description which follows, the embodiments may be taken alone or combined appropriately by those skilled in the art.

### Mutant polypeptide

In the context of the present invention, the Inventors have developed novel phloroglucinol synthases (Phld) with optimised activity, that are particularly suitable for large industrial-scale production.

The Inventors designed an original and comprehensive mutagenesis strategy to identify mutant polypeptides from the phloroglucinol synthase of the actinomycete bacteria *Tsukamurella pulmonis* (Phld_Tpu, wild type (WT)), with improved properties, including increased phloroglucinol synthase activity, increased substrate affinity.

In particular, state-of-the-art computational studies were carried out to predict Phld_Tpu three-dimensional structure in its functional homodimeric form and to uncover molecular determinants involved in substrate and product recognition. Using these original computer-aided strategies, several amino acid positions were predicted as targets for mutagenesis to improve catalytic activity and stability of the enzyme. Several libraries of mutants were then constructed and screened for improved Phlo production.

This combination of complementary strategies led to the identification of several simple mutants, as well as mutants with double or even multiple mutations, having a Phlo activity significantly higher compared to WT Phld_Tpu. Indeed, the novel phloroglucinol synthase mutants allow to obtain concentrations of phloroglucinol up to 90% higher than those obtained with the wild type Phld (i.e., the Inventors were able to obtain concentrations of phloroglucinol of up to 190% of concentrations of phloroglucinol obtained using the wild type). The Inventors unexpectedly identified several key amino acid residues, allowing optimisation of the enzymatic activity.

The Inventors demonstrate here that these mutant phloroglucinol synthases are functional when they are produced in a prokaryotic system as well as a eukaryotic system and are suitable for large industrial-scale production.

The present invention thus concerns a mutant polypeptide comprising, or consisting essentially of, or consisting of, an amino acid sequence of a phloroglucinol synthase having at least 80% of identity with the amino acid sequence of the *Tsukamurella pulmonis* phloroglucinol synthase as set forth in SEQ ID NO: 1, preferably an amino acid sequence of a phloroglucinol synthase having at least 81 % of identity with the amino acid sequence of the *Tsukamurella pulmonis* phloroglucinol synthase as set forth in SEQ ID NO: 1, preferably an amino acid sequence of a phloroglucinol synthase having at least 82 % of identity, preferably an amino acid sequence of a phloroglucinol synthase having at least 83 % of identity, preferably an amino acid sequence of a phloroglucinol synthase having at least 84 % of identity, preferably an amino acid sequence of a phloroglucinol synthase having at least 85 % of identity, preferably an amino acid sequence of a phloroglucinol synthase having at least 86 % of identity, preferably an amino acid sequence of a phloroglucinol synthase having at least 87 % of identity, preferably an amino acid sequence of a phloroglucinol synthase having at least 88 % of identity, preferably an amino acid sequence of a phloroglucinol synthase having at least 89 % of identity, preferably an amino acid sequence of a phloroglucinol synthase having at least 90 % of identity, preferably an amino acid sequence of a phloroglucinol synthase having at least 91 % of identity, preferably an amino acid sequence of a phloroglucinol synthase having at least 92 % of identity, preferably an amino acid sequence of a phloroglucinol synthase having at least 93 % of identity, preferably an amino acid sequence of a phloroglucinol synthase having at least 94 % of identity, preferably an amino acid sequence of a phloroglucinol synthase having at least 95 % of identity, preferably an amino acid sequence of a phloroglucinol synthase having at least 96 % of identity, preferably an amino acid sequence of a phloroglucinol synthase having at least 97 % of identity, preferably an amino acid sequence of a phloroglucinol synthase having at least 98 % of identity, preferably an amino acid sequence of a phloroglucinol synthase having at least 99 % of identity, with the amino acid sequence of the *Tsukamurella pulmonis* phloroglucinol synthase as set forth in SEQ ID NO: 1, even more preferably an amino acid sequence of a phloroglucinol synthase having the amino acid sequence of the *Tsukamurella pulmonis* phloroglucinol synthase as set forth in SEQ ID NO: 1;
said amino acid sequence of a phloroglucinol synthase having one or more substitution(s) of one or more amino acid residue(s) selected from the group consisting of:
a) any amino acid residue or combination of amino acid residues located from position 268 to position 271 of SEQ ID NO:1; or
   any amino acid residue or combination of amino acid residues located from a position equivalent to position 268 to a position equivalent to position 271 of SEQ ID NO: 1, after optimal global alignment with SEQ ID NO:1; or
b) an amino acid residue located at position 49 of SEQ ID NO:1; or
   an amino acid residue located at a position equivalent to position 49 of SEQ ID NO:1, after optimal global alignment with SEQ ID NO:1; or
c) an amino acid residue located at position 339 of SEQ ID NO:1; or
   an amino acid residue located at a position equivalent to position 339 of SEQ ID NO:1, after optimal global alignment with SEQ ID NO:1; or
d) amino acid residues located at positions 93 and 262 of SEQ ID NO:1; or
   amino acid residues located at positions equivalent to positions 93 and 262 of SEQ ID NO:1, after optimal global alignment with SEQ ID NO:1; or
e) amino acid residues located at positions 129 and 176 of SEQ ID NO:1; or
   amino acid residues located at positions equivalent to positions 129 and 176 of SEQ ID NO:1, after optimal global alignment with SEQ ID NO:1; and
f) any combination thereof.

Preferably, the present invention may concern a mutant polypeptide comprising, or consisting essentially of, or consisting of, an amino acid sequence of a phloroglucinol synthase having at least 80 % of identity with the amino acid sequence of the *Tsukamurella pulmonis* phloroglucinol synthase as set forth in SEQ ID NO: 1, preferably an amino acid sequence of a phloroglucinol synthase having at least 81 % of identity with the amino acid sequence of the *Tsukamurella pulmonis* phloroglucinol synthase as set forth in SEQ ID NO: 1, preferably an amino acid sequence of a phloroglucinol synthase having at least 82 % of identity, preferably an amino acid sequence of a phloroglucinol synthase having at least 83 % of identity, preferably an amino acid sequence of a phloroglucinol synthase having at least 84 % of identity, preferably an amino acid sequence of a phloroglucinol synthase having at least 85 % of identity, preferably an amino acid sequence of a phloroglucinol synthase having at least 86 % of identity, preferably an amino acid sequence of a phloroglucinol synthase having at least 87 % of identity, preferably an amino acid sequence of a phloroglucinol synthase having at least 88 % of identity, preferably an amino acid sequence of a phloroglucinol synthase having at least 89 % of identity, preferably an amino acid sequence of a phloroglucinol synthase having at least 90 % of identity, preferably an amino acid sequence of a phloroglucinol synthase having at least 91 % of identity, preferably an amino acid sequence of a phloroglucinol synthase having at least 92 % of identity, preferably an amino acid sequence of a phloroglucinol synthase having at least 93 % of identity, preferably an amino acid sequence of a phloroglucinol synthase having at least 94 % of identity, preferably an amino acid sequence of a phloroglucinol synthase having at least 95 % of identity, preferably an amino acid sequence of a phloroglucinol synthase having at least 96 % of identity, preferably an amino acid sequence of a phloroglucinol synthase having at least 97 % of identity, preferably an amino acid sequence of a phloroglucinol synthase having at least 98 % of identity, preferably an amino acid sequence of a phloroglucinol synthase having at least 99 % of identity, with the amino acid sequence of the *Tsukamurella pulmonis* phloroglucinol synthase as set forth in SEQ ID NO: 1, even more preferably an amino acid sequence of a phloroglucinol synthase having the amino acid sequence of the *Tsukamurella pulmonis* phloroglucinol synthase as set forth in SEQ ID NO: 1;
said amino acid sequence of a phloroglucinol synthase having one or more substitution(s) of one or more amino acid residue(s) selected from the group consisting of:
- any amino acid residue or combination of amino acid residues located from position 268 to position 271 of SEQ ID NO:1; or
- any amino acid residue or combination of amino acid residues located from a position equivalent to position 268 to a position equivalent to position 271 of SEQ ID NO: 1, after optimal global alignment with SEQ ID NO:1.

As detailed in the section above entitled "Definition", the percent identities referred to in the context of the present invention are determined on the basis of an optimal global alignment of sequences to be compared (as defined above).

Once an optimal global alignment has been obtained between SEQ ID NO:1 and the other sequence (also called "the other sequence" or "the sequence to align with SEQ ID NO:1"), the percentage of identity and position equivalent to specific position(s) of SEQ ID NO:1 are defined. In particular, a position equivalent to position X of SEQ ID NO:1 is the position of the other sequence that is aligned with position X of SEQ ID NO:1.

The score (that may be referred to as a "similarity score" as explained in the section above entitled "Definition") may also be determined as explained in the section above entitled "Definition", after optimal global alignment of SEQ ID NO:1 and the other sequence (or "the sequence to align with SEQ ID NO:1"). The higher is the value of the score between SEQ ID NO:1 and the other sequence, the higher is the similarity between this other sequence and SEQ ID NO:1, taking into account not only aligned identical amino acids, but also conservative substitutions.

In a preferred embodiment, the mutant polypeptide comprises one or more substitutions of any amino acid residue or combination of amino acid residues located from position 268 to position 271 of SEQ ID NO:1, or comprises one or more substitutions of any amino acid residue or combination of amino acid residues located from a position equivalent to position 268 to a position equivalent to position 271 of SEQ ID NO: 1, after optimal global alignment with SEQ ID NO:1. Indeed, the data obtained by the Inventors demonstrate that the Phlo activity is particularly elevated for mutants comprising a substitution in this zone.

Advantageously, the mutant polypeptide, comprises:
a) a substitution of the glutamic acid (E) residue located at position 271 of SEQ ID NO:1, or of the amino acid residue located at a position equivalent to position 271 of SEQ ID NO: 1 after optimal global alignment with SEQ ID NO:1, preferably wherein the glutamic acid residue located at position 271 of SEQ ID NO:1, or the amino acid residue located at a position equivalent to position 271 of SEQ ID NO: 1 after optimal global alignment with SEQ ID NO:1, is substituted by an amino acid residue selected from the group consisting of arginine (R), histidine (H), lysine (K), glutamine (Q), valine (V), methionine (M), phenylalanine (F), leucine (L), tyrosine (Y), and proline (P); preferably substituted by an amino acid residue selected from the group consisting of arginine (R), histidine (H), lysine (K), valine (V), methionine (M), and phenylalanine (F) ; more preferably substituted by an amino acid residue selected from the group consisting of arginine (R), lysine (K), valine (V), and methionine (M); even more preferably substituted by an amino acid residue selected from the group consisting of arginine (R), lysine (K), and valine (V); even more preferably substituted by an amino acid residue selected from the group consisting of arginine (R), and lysine (K);
b) substitutions of the glutamic acid (E) residues located at positions 268 and 271 of SEQ ID NO:1, or of the amino acid residues located at positions equivalent to positions 268 and 271 of SEQ ID NO: 1 after optimal global alignment with SEQ ID NO:1, preferably wherein:
   - the glutamic acid (E) residue located at position 268 of SEQ ID NO:1, or the amino acid residue located at a position equivalent to position 268 of SEQ ID NO: 1 after optimal global alignment with SEQ ID NO:1, is substituted by an amino acid residue selected from Q, T, and S; and
   - the glutamic acid (E) residue located at position 271 of SEQ ID NO:1, or the amino acid residue located at a position equivalent to position 271 of SEQ ID NO: 1 after optimal global alignment with SEQ ID NO:1, is substituted by an amino acid residue selected from K, R and V; or
c) substitutions of amino acid residues located at positions 124, 154, 177, 236, 268, 271 and 298 of SEQ ID NO:1 or of amino acid residues located at positions equivalent to positions 124, 154, 177, 236, 268, 271 and 298 of SEQ ID NO:1, preferably wherein:
   - the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
   - the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
   - the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A); and
   - the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
   - the glutamic acid (E) residue located at position 268 of SEQ ID NO:1, or the amino acid residue located at a position equivalent to position 268 of SEQ ID NO: 1 after optimal global alignment with SEQ ID NO:1, is substituted by an amino acid residue selected from Q, T, and S; and
   - the glutamic acid (E) residue located at position 271 of SEQ ID NO:1, or the amino acid residue located at a position equivalent to position 271 of SEQ ID NO: 1 after optimal global alignment with SEQ ID NO:1, is substituted by an amino acid residue selected from K, R and V; and
   - the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue; or
d) substitutions of amino acid residues located at positions 124, 154, 177, 236, 268, 270, 271 and 298 of SEQ ID NO:1 or of amino acid residues located at positions equivalent to positions 124, 154, 177, 236, 268, 270, 271 and 298 of SEQ ID NO:1, preferably wherein:
   - the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
   - the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
   - the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A); and
   - the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
   - the glutamic acid (E) residue located at position 268 of SEQ ID NO:1, or the amino acid residue located at a position equivalent to position 268 of SEQ ID NO: 1 after optimal global alignment with SEQ ID NO:1, is substituted by an amino acid residue selected from Q, T, and S; and
   - the alanine (A) residue located at position 270 of SEQ ID NO:1, or the amino acid residue located at a position equivalent to position 270 of SEQ ID NO: 1 after optimal global alignment with SEQ ID NO:1, is substituted by a serine (S) residue; and
   - the glutamic acid (E) residue located at position 271 of SEQ ID NO:1, or the amino acid residue located at a position equivalent to position 271 of SEQ ID NO: 1 after optimal global alignment with SEQ ID NO:1, is substituted by an amino acid residue selected from K, R and V; and
   - the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue; or
e) substitutions of amino acid residues located at positions 35, 60, 91, 123, 124, 154, 177, 236, 270, and 298 of SEQ ID NO:1 or of amino acid residues located at positions equivalent to positions 35, 60, 91, 123, 124, 154, 177, 236, 270, and 298 of SEQ ID NO:1, preferably wherein:
   - the serine (S) residue located at position 35 of SEQ ID NO:1, or the amino acid residue located at a position equivalent to position 35 of SEQ ID NO: 1 after optimal global alignment with SEQ ID NO:1, is substituted by a glycine (G) residue; and
   - the alanine (A) residue located at position 60 of SEQ ID NO:1, or the amino acid residue located at a position equivalent to position 60 of SEQ ID NO: 1 after optimal global alignment with SEQ ID NO:1, is substituted by an arginine (R) residue; and
   - the alanine (A) residue located at position 91 of SEQ ID NO:1, or the amino acid residue located at a position equivalent to position 91 of SEQ ID NO: 1 after optimal global alignment with SEQ ID NO:1, is substituted by an arginine (R) residue; and
   - the alanine (A) residue located at position 123 of SEQ ID NO:1, or the amino acid residue located at a position equivalent to position 123 of SEQ ID NO: 1 after optimal global alignment with SEQ ID NO:1, is substituted by a serine (S) residue; and
   - the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
   - the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
   - the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A) residue; and
   - the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
   - the alanine (A) residue located at position 270 of SEQ ID NO:1, or the amino acid residue located at a position equivalent to position 270 of SEQ ID NO: 1 after optimal global alignment with SEQ ID NO:1, is substituted by a serine (S) residue; and
   - the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue; or
f) substitutions of amino acid residues located at positions 35, 46, 60, 91, 123, 124, 154, 177, 191, 236, 238, 270, 290 and 298 of SEQ ID NO:1 or of amino acid residues located at positions equivalent to positions 35, 46, 60, 91, 123, 124, 154, 177, 191, 236, 238, 270, 290 and 298 of SEQ ID NO:1, preferably wherein:
   - the serine (S) residue located at position 35 of SEQ ID NO:1, or the amino acid residue located at a position equivalent to position 35 of SEQ ID NO: 1 after optimal global alignment with SEQ ID NO:1, is substituted by a glycine (G) residue; and
   - the serine (S) residue located at position 46 of SEQ ID NO:1, or the amino acid residue located at a position equivalent to position 46 of SEQ ID NO: 1 after optimal global alignment with SEQ ID NO:1, is substituted by an alanine (A) residue; and
   - the alanine (A) residue located at position 60 of SEQ ID NO:1, or the amino acid residue located at a position equivalent to position 60 of SEQ ID NO: 1 after optimal global alignment with SEQ ID NO:1, is substituted by an arginine (R) residue; and
   - the alanine (A) residue located at position 91 of SEQ ID NO:1, or the amino acid residue located at a position equivalent to position 91 of SEQ ID NO: 1 after optimal global alignment with SEQ ID NO:1, is substituted by an arginine (R) residue; and
   - the alanine (A) residue located at position 123 of SEQ ID NO:1, or the amino acid residue located at a position equivalent to position 123 of SEQ ID NO: 1 after optimal global alignment with SEQ ID NO:1, is substituted by a serine (S) residue; and
   - the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
   - the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
   - the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A) residue; and
   - the methionine (M) residue located at position 191 of SEQ ID NO:1, or the amino acid residue located at a position equivalent to position 191 of SEQ ID NO: 1 after optimal global alignment with SEQ ID NO:1, is substituted by a valine (V) residue; and
   - the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
   - the glutamine (Q) residue located at position 238 of SEQ ID NO:1, or the amino acid residue located at a position equivalent to position 238 of SEQ ID NO: 1 after optimal global alignment with SEQ ID NO:1, is substituted by a lysine (K) residue; and
   - the alanine (A) residue located at position 270 of SEQ ID NO:1, or the amino acid residue located at a position equivalent to position 268 of SEQ ID NO: 1 after optimal global alignment with SEQ ID NO:1, is substituted by a serine (S) residue; and
   - the aspartic acid (D) residue located at position 290 of SEQ ID NO:1, or amino acid located at a position equivalent to position 290 of SEQ ID NO: 1 after optimal global alignment with SEQ ID NO:1, is substituted by an alanine (A) residue; and
   - the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue.

The mutant polypeptide preferably comprises, or consists essentially of, or consists of, the amino acid sequence as defined in SEQ ID NO:2:
MNTTEQQSVLPQQAWLGAPPAPPTSVAVIESLATX₃₅SPTQTYGQAEX₄₆ADX₄₉VAARFDDPRQX₆₀ERI RRVYAKTRVTERHLAIDPLTPEFAEFSX₉₁RX₉₃DTVRERMDLFFEHAAPLAIETARRALGDNX₁₂₃X₁₂₄TDI GX₁₂₉LVFVTSTGFLAPGVDVAVIRALGLX₁₅₄PQTSRVVINFMGCAAAMNALRX₁₇₆X₁₇₇TDYVRAQPDRK SLX₁₉₁ICLELSSVNAVFSGDPNDVVISSLFADGCGAAVIGASEVGHPLPX₂₃₆GX₂₃₈IVVRDTFTHLLDGAE DGIVLGVNX₂₆₂NGITCX₂₆₈LX₂₇₀X₂₇₁SLPQYIVNGVAPVVDAVLX₂₉₀RNGLGREX₂₉₈VAHWAIHPGGPK IIESAAAALGLPDDASRLSWDVLAEHGNX₃₃₉LSVSLLFVLERLRAQVASDGADGAPSTGMAFSFAPGVT VEGFLFDVVTTGE;
wherein :
a) the amino acid residue X₂₇₁ is any amino acid residue selected from the group consisting of arginine (R), histidine (H), lysine (K), glutamine (Q), valine (V), methionine (M), phenylalanine (F), leucine (L), tyrosine (Y), and proline (P); preferably the amino acid residue X₂₇₁ is selected from the group consisting of arginine (R), histidine (H), lysine (K), valine (V), methionine (M), and phenylalanine (F) ; more preferably the amino acid residue X₂₇₁ is selected from the group consisting of arginine (R), lysine (K), valine (V), and methionine (M), even more preferably the amino acid residue X₂₇₁ is selected from the group consisting of arginine (R), lysine (K), and valine (V);
   and
b) the amino acid residues X₃₅, X₄₆, X₄₉, X₆₀, X₉₁, X₉₃, X₁₂₃, X₁₂₄, X₁₂₉, X₁₅₄, X₁₇₆, X₁₇₇, X₁₉₁, X₂₃₆, X₂₃₈, X₂₆₂, X₂₆₈, X₂₇₀, X₂₉₀, X₂₉₈, and X₃₃₉ are independently selected from any amino acid ; preferably wherein :
   i. the amino acid residue X₃₅ is the amino acid residue S or G, preferably G;
   ii. the amino acid residue X₄₆ is the amino acid residue 5 or A, preferably A;
   iii. the amino acid residue X₄₉ is any amino acid residue selected from the group consisting of R, A and S, preferably X₄₉ is any amino acid residue A or S;
   iv. the amino acid residue X₆₀ is the amino acid residue A or R, preferably R;
   v. the amino acid residue X₉₁ is the amino acid residue A or R, preferably R;
   vi. the amino acid residue X₉₃ is the amino acid residue P or C, preferably C;
   vii. the amino acid residue X₁₂₃ is the amino acid residue A or S, preferably S;
   viii. the amino acid residue X₁₂₄ is the amino acid residue A or P, preferably P;
   ix. the amino acid residue X₁₂₉ is the amino acid residue Q or M, preferably M;
   x. the amino acid residue X₁₅₄ is the amino acid residue A or P, preferably P;
   xi. the amino acid residue X₁₇₆ is any amino acid residue selected from the group consisting of V, I and L; preferably X₁₇₆ is L or I, preferably I;
   xii. the amino acid residue X₁₇₇ is the amino acid residue 5 or A, preferably A;
   xiii. the amino acid residue X₁₉₁ is the amino acid residue M or V, preferably V;
   xiv. the amino acid residue X₂₃₆ is the amino acid residue S or P, preferably P;
   xv. the amino acid residue X₂₃₈ is the amino acid residue Q or K, preferably K;
   xvi. the amino acid residue X₂₆₂ is the amino acid residue A or C, preferably C;
   xvii. the amino acid residue X₂₆₈ is any amino acid residue selected from the group consisting of E, Q, T, S, C, G, K, M, and R; preferably X₂₆₈ is any amino acid residue selected from the group consisting of Q, T, S, C, G, K, M, and R; preferably X₂₆₈ is any amino acid residue selected from the group consisting of Q, T, and S;
   xviii. the amino acid residue X₂₇₀ is the amino acid residue A or S, preferably S;
   xix. the amino acid residue X₂₉₀ is the amino acid residue D or A, preferably A;
   xx. the amino acid residue X₂₉₈ is the amino acid residue A or D, preferably D;
   xxi. the amino acid residue X₃₃₉ is any amino acid residue selected from the group consisting of M, I, Q, and T; preferably X₃₃₉ is any amino acid residue selected from the group consisting of I, Q, and T, preferably X₃₃₉ is I; or
   xxii. any combination thereof.

In a preferred embodiment, the mutant polypeptide comprises, or consists essentially of, or consists of, the amino acid sequence set forth in SEQ ID NO: 1, with one or more substitution(s) (with one substitution or a group of substitutions) selected from the group consisting of:
1a. A124P, A154P, S177A, S236P, E268Q, E271R, and A298D (mutant polypeptide M27);
2a. A124P, A154P, S177A, S236P, E268T, E271K, and A298D (mutant polypeptide M28);
3a. A124P, A154P, S177A, S236P, E268S, A270S, E271K, and A298D;
4a. A124P, A154P, S177A, S236P, E268Q, E271K, and A298D;
5a. A124P, A154P, S177A, S236P, E268S, E271R, and A298D;
6a. E268Q and E271K;
7a. E268T and E271K;
8a. E268T and E271R;
9a. A124P, A154P, S177A, S236P, E268Q, A270S, E271K, and A298D;
10a. A124P, A154P, S177A, S236P, E268T, A270S, E271R, and A298D;
11a. A124P, A154P, S177A, S236P, E268T, A270S, E271V, and A298D;
12a. A124P, A154P, S177A, S236P, E268S, E271K, and A298D;
13a. Q129M and V176I;
14a. E268Q and E271R (mutant polypeptide M29);
15a. A124P, A154P, S177A, S236P, E268Q, A270S, E271R, and A298D;
16a. A124P, A154P, S177A, S236P, E268S, A270S, E271R, and A298D;
17a. E271K;
18a. S35G, A60R, A91R, A123S, A124P, A154P, S177A, S236P, A270S, and A298D (mutant polypeptide M22);
19a. E268S and E271K;
20a. P93C and A262C;
21a. E268T and E271V;
22a. A124P, A154P, S177A, S236P, E268T, A270S, E271K, and A298D;
23a. E271R;
24a. S35G, S46A, A60R, A91R, A123S, A124P, A154P, S177A, M191V, S236P, Q238K, A270S, D290A, and A298D (mutant polypeptide M23);
25a. R49A; and
26a. M339I.

In a preferred embodiment, the mutant polypeptide comprises, or consists essentially of, or consists of, the amino acid sequence set forth in SEQ ID NO: 1, with one or more substitution(s) (with one substitution or a group of substitutions) selected from the group consisting of:
1b. A124P, A154P, S177A, S236P, E268Q, E271R, and A298D (mutant polypeptide M27);
2b. A124P, A154P, S177A, S236P, E268T, E271K, and A298D (mutant polypeptide M28);
3b. A124P, A154P, S177A, S236P, E268S, A270S, E271K, and A298D;
4b. A124P, A154P, S177A, S236P, E268Q, E271K, and A298D;
5b. A124P, A154P, S177A, S236P, E268S, E271R, and A298D;
6b. E268Q and E271K;
7b. E268T and E271K;
8b. E268T and E271R;
9b. A124P, A154P, S177A, S236P, E268Q, A270S, E271K, and A298D;
10b. A124P, A154P, S177A, S236P, E268T, A270S, E271R, and A298D;
11b. A124P, A154P, S177A, S236P, E268T, A270S, E271V, and A298D;
12b. A124P, A154P, S177A, S236P, E268S, E271K, and A298D;
13b. E268Q and E271R (mutant polypeptide M29);
14b. A124P, A154P, S177A, S236P, E268Q, A270S, E271R, and A298D;
15b. A124P, A154P, S177A, S236P, E268S, A270S, E271R, and A298D;
16b. E271K;
17b. S35G, A60R, A91R, A123S, A124P, A154P, S177A, S236P, A270S, and A298D (mutant polypeptide M22);
18b. E268S and E271K;
19b. E268T and E271V;
20b. A124P, A154P, S177A, S236P, E268T, A270S, E271K, and A298D;
21b. E271R;
22b. S35G, S46A, A60R, A91R, A123S, A124P, A154P, S177A, M191V, S236P, Q238K, A270S, D290A, and A298D (mutant polypeptide M23).

Indeed, the Inventors showed that these mutants are particularly optimized, with improved properties, including increased phloroglucinol synthase activity.

Advantageously, the mutant polypeptide comprises, or consists essentially of, or consists of, an amino acid sequence selected from the group consisting of SEQ ID NO: 3-28.

According to one embodiment, the mutant polypeptide comprises, or consists essentially of, or consists of, at least one amino acid sequence preferably having at least 60% identity, more preferably at least 65% identity, more preferably at least 70% identity, even more preferably at least 75% identity, still more preferably at least 80% identity, even more preferentially at least 85% identity, even more preferentially at least 90% identity, more preferably at least 95% identity, more preferably at least 96% identity, even more preferably at least 97% identity, still more preferably at least 98% identity, and even more preferentially at least 99% identity, with an amino acid sequence selected from the group consisting of SEQ ID NO: 3-28.

The mutant polypeptide has preferably a Phld activity higher compared to the wild-type polypeptide (i.e. the unmutated polypeptide, preferably the polypeptide of SEQ ID NO:1). In an advantageous embodiment, the mutant polypeptide allows to obtain a concentration of phloroglucinol, at least 10% higher than those obtained with the wild-type polypeptide (i.e. the unmutated polypeptide, preferably the polypeptide of SEQ ID NO:1), using the same conditions. In a more advantageous embodiment, the mutant polypeptide allows to obtain a concentration of phloroglucinol at least 15% higher (preferably at least 20% higher, preferably at least 25% higher, preferably at least 30% higher, preferably at least 35% higher, preferably at least 40% higher, preferably at least 45% higher, preferably at least 50% higher, preferably at least 55% higher, preferably at least 60% higher, preferably at least 65% higher, at least 70% higher, preferably at least 75% higher, preferably at least 80% higher, preferably at least 85% higher, preferably at least 90% higher, preferably at least 95% higher, even more preferably at least 100% higher) than those obtained with the wild-type polypeptide (i.e. the unmutated polypeptide, preferably the polypeptide of SEQ ID NO:1), using the same conditions (e.g., with the same experimental settings).

In other words, the mutant polypeptide advantageously allows to obtain a concentration of phloroglucinol of at least 110% of the concentration of phloroglucinol obtained using the wild-type polypeptide (i.e. the unmutated polypeptide, preferably the polypeptide of SEQ ID NO:1), with the same experimental settings. Preferably, the mutant polypeptide allows to obtain a concentration of phloroglucinol of at least 115% (preferably at least 120%, preferably at least 125%, preferably at least 130%, preferably at least 135%, preferably at least 140%, preferably at least 145%, preferably at least 150%, preferably at least 155%, preferably at least 160%, preferably at least 165%, at least 170%, preferably at least 175%, preferably at least 180%, preferably at least 185%, preferably at least 190%, preferably at least 195%, even more preferably at least 200%) of the concentration of phloroglucinol obtained using the wild-type polypeptide (i.e. the unmutated polypeptide, preferably the polypeptide of SEQ ID NO:1), with the same experimental settings.

Thus, the mutant polypeptide preferably performs Phlo synthesis with an efficiency of at least 110% (preferably at least 115, preferably at least 120%, preferably at least 125%, preferably at least 130%, preferably at least 135%, preferably at least 140%, preferably at least 145%, preferably at least 150%, preferably at least 155%, preferably at least 160%, preferably at least 165%, at least 170%, preferably at least 175%, preferably at least 180%, preferably at least 185%, preferably at least 190%, preferably at least 195%, even more preferably at least 200%) of that of the wild-type polypeptide (i.e. the unmutated polypeptide, preferably the polypeptide of SEQ ID NO:1), in the same experimental settings.

In some embodiments, the mutant polypeptide further comprises one or more additional mutation(s), such as mutation(s) selected from the group consisting of substitution, deletion, and insertion; preferably wherein the one or more additional mutation(s), is(are) conservative mutation(s), in particular when the one or more additional mutation(s) is(are) substitution(s). The nature of the mutations introduced can indeed influence the activity of the resulting mutant and it is well known that conservative substitutions are less likely to alter the enzymatic activity of a mutant than non-conservative substitutions.

Preferably, when the mutant polypeptide further comprises one or more additional mutation(s), the mutant polypeptide retains Phlo activity, at least partially. Thus, the mutant polypeptide further comprising one or more additional mutation(s), preferably performs Phlo synthesis with an efficiency of at least 100% (preferably at least 101%, preferably at least 102%, preferably at least 103%, preferably at least 104%, more preferably at least 105%, more preferably at least 110%, more preferably at least 115%, more preferably at least 120%) of that of the wild-type polypeptide (i.e. the unmutated polypeptide, preferably the polypeptide of SEQ ID NO:1), in the same experimental settings. In other word, the mutant polypeptide further comprising one or more additional mutation(s) allows to obtain a concentration of phloroglucinol, at least equal to those obtained with the wild-type polypeptide (i.e. the unmutated polypeptide, preferably the polypeptide of SEQ ID NO:1), using the same conditions. In a more advantageous embodiment, the mutant polypeptide further comprising one or more additional mutation(s) allows to obtain a concentration of phloroglucinol at least 1% higher (preferably at least 2% higher, preferably at least 3% higher, preferably at least 4% higher, preferably at least 5% higher, preferably at least 10% higher, preferably at least 15% higher, more preferably at least 20% higher) than those obtained with the wild-type polypeptide (i.e. the unmutated polypeptide, preferably the polypeptide of SEQ ID NO:1), using the same conditions (e.g., with the same experimental settings). Thus, the mutant polypeptide further comprising one or more additional mutation(s) advantageously allows to obtain a concentration of phloroglucinol of at least 100% (preferably at least 101%, preferably at least 102%, preferably at least 103%, preferably at least 104%, more preferably at least 105%, more preferably at least 110%, more preferably at least 115%, more preferably at least 120%) of the concentration of phloroglucinol obtained using the wild type polypeptide (i.e. the unmutated polypeptide, preferably the polypeptide of SEQ ID NO:1), with the same experimental settings.

The mutant polypeptide may advantageously further comprise an insertion, of a single amino acid residue, a peptide, or a polypeptide, and any combination thereof.

The present invention thus also relates to a fusion polypeptide comprising, or consisting essentially of, or consisting of, the mutant polypeptide of the invention, and an additional peptide, polypeptide or protein. Said additional peptide, polypeptide or protein may be inserted:
a) at the N-terminus of the mutant polypeptide (i.e., in N-terminal position), and/or
b) at the C-terminus of the mutant polypeptide (i.e., in C-terminal position), and/or
c) between any two consecutive amino acid residues of the mutant polypeptide.

Said additional peptide, polypeptide or protein may have various functions. A useful function is a tag permitting identification and/or purification of the fusion. It is indeed also known that the addition of a tag peptide or polypeptide, which makes the mutant easier to detect or purify, generally does not alter the enzymatic activity, especially when the tag peptide or polypeptide is inserted in N- or C-terminal (rather than inside the sequence).

When the additional peptide, polypeptide or protein is inserted at the N-terminus and/or at the C-terminus of the mutant polypeptide, it may be fused directly to the N-terminal extremity and/or to the C-terminal extremity of the mutant polypeptide, or separated from the N-terminal extremity and/or from the C-terminal extremity of the mutant polypeptide by a linker peptide.

Typically, linkers are 1 to 30 amino acids long peptides composed of amino acid residues such as glycine (G), serine (S), threonine (T), asparagine (N), glutamine (Q), alanine (A) proline (P), and/or phenylalanine (F). Preferred linkers in the context of this invention comprise 2 to 15 amino acids, with a preference for 2, 3, 4, 5, 6, 7, 8, 9, 10, 11 or 12 amino acids.

### Nucleic acid molecule

The present invention also relates to a nucleic acid molecule comprising a nucleic acid sequence encoding a mutant polypeptide according to the invention, preferably wherein:
a) the nucleic acid molecule is isolated; or
b) the nucleic acid molecule further comprises a promoter controlling expression of the nucleic acid sequence; or
c) the isolated nucleic acid molecule further comprises a transcription terminator controlling expression of the nucleic acid sequence; or
d) the nucleic acid sequence is further optimized for expression in a host cell, in particular a yeast or a bacterium; or
e) any combination of a) to d).

Advantageously, the mutant polypeptide is any mutant polypeptide as defined above in section "Mutant polypeptide".

Such nucleic acid molecules are useful either for the production of a mutant polypeptide as defined above, or for the direct production of phloroglucinol when introduced or transfected (directly or within a vector) into a host cell, cultured in the presence of a suitable substrate.

Advantageously, the nucleic acid molecule comprises, or consists essentially of, or consists of, a nucleic acid sequence selected from the group consisting of SEQ ID NO: 46-72.

According to one embodiment, the nucleic acid molecule comprises, or consists essentially of, or consists of, at least one nucleic acid sequence preferably having at least 60% identity, more preferably at least 65% identity, more preferably at least 70% identity, even more preferably at least 75% identity, still more preferably at least 80% identity, even more preferentially at least 85% identity, even more preferentially at least 90% identity, more preferably at least 95% identity, more preferably at least 96% identity, even more preferably at least 97% identity, still more preferably at least 98% identity, and even more preferentially at least 99% identity, with a nucleic acid sequence selected from the group consisting of SEQ ID NO: 46-72.

In some embodiments, the nucleic acid molecule comprises one or more additional mutation(s), such as mutation(s) selected from the group consisting of substitution, deletion, and insertion; preferably wherein the one or more additional mutation(s), is(are) silent mutation(s), in particular when the one or more additional mutation(s) is(are) substitution(s). The nature of the mutations introduced can indeed influence the activity of the resulting mutant and it is well known that silent substitutions do not alter the enzymatic activity of a mutant.

Advantageously, in the nucleic acid molecule according to the invention :
a) when the nucleic acid molecule further comprises a promoter controlling the expression of the nucleic acid sequence, the promoter is an exogenous promoter, in particular a yeast promoter; or
b) when the isolated nucleic acid molecule further comprises a transcription terminator controlling the expression of the nucleic acid sequence, the transcription terminator is an exogenous terminator, such as a yeast terminator; or
c) the nucleic acid sequence is further optimized for expression in yeast.

According to one embodiment, the nucleic acid molecule comprises a promoter controlling the expression of at least one nucleic acid sequence encoding a mutant polypeptide as defined above.

Advantageously, the promoter is an exogenous promoter, in particular a yeast promoter, preferably a promoter chosen from ADH2 promoter (pADH2), CCW12 promoter (pCCW12), TEF1 promoter (pTEF1), PGK1 promoter (pPGK1), TDH3 promoter (pTDH3), TPI1 promoter (pTPI1), more preferably a promoter chosen from ADH2 promoter (pADH2) of *Saccharomyces cerevisiae,* CCW12 promoter of S. *cerevisiae,* TEF1 promoter of S. *cerevisiae,* PGK1 promoter of S. cerevisiae, TDH3 promoter of S. *cerevisiae,* and TPI1 promoter of S. *cerevisiae;* more preferably a promoter chosen from ADH2 promoter of SEQ ID NO: 29, CCW12 promoter of SEQ ID NO: 30, TEF1 promoter of SEQ ID NO: 31, PGK1 promoter of SEQ ID NO:73, TDH3 promoter of SEQ ID NO:74, and TPI1 promoter of SEQ ID NO:75, and in particular a promoter chosen from ADH2 promoter of SEQ ID NO: 29, CCW12 promoter of SEQ ID NO: 30, and TEF1 promoter of SEQ ID NO: 31.

According to one embodiment, the nucleic acid molecule comprises a transcription terminator for at least one nucleic acid sequence encoding a mutant polypeptide as defined above.

Advantageously, the terminator is an exogenous terminator, in particular a yeast terminator, preferably the RPL3 terminator (tRPL3), the ADH1 terminator (tADH1) or the CYC1 terminator (tCYC1); more preferably the RPL3 terminator of S. *cerevisiae,* or the ADH1 terminator of S. *cerevisiae;* more preferably the RPL3 terminator of SEQ ID NO: 32, the ADH1 terminator of SEQ ID NO: 33, or the CYC1 terminator of SEQ ID NO:76, and in particular the RPL3 terminator of SEQ ID NO: 32 or the ADH1 terminator of SEQ ID NO: 33.

According to one preferred embodiment, the nucleic acid molecule comprises both a promoter and a terminator which are as defined above.

According to one embodiment, the nucleic acid molecule also comprises an export sequence. Advantageously, this export sequence allows the secretion or excretion of the polypeptide(s) encoded by said nucleic acid molecule, in the cell medium.

According to one preferred embodiment, the nucleic acid sequence is further optimized for expression in a host cell, in particular a yeast or a bacterium. To optimize expression in a host cell, in particular in yeast or bacteria, those skilled in the art know how to use genetic code usage bias, which designates the preferential use for a given organism of one of the possible triplets of nucleotides or codons to code for the same amino acid. Indeed, there are usually several combinations of three nucleotides (called "codons") coding for the same amino acid (except for methionine and tryptophan), called synonymous codons, but some of these combinations are generally used preferentially by a given organism. For the production of an amino acid sequence of interest, optimal expression can thus be achieved when the codons chosen to encode the amino acid sequence are those used preferentially by the host cell's organism of origin. Depending on the selected production organism (notably yeast or bacteria), different optimal nucleic acid sequences will therefore be used when the nucleic acid sequence comprised in the isolated nucleic acid molecule used in the invention is further optimized for expression in the selected production organism. Various software packages are available to the person in the trade to optimize codons for expression in a host cell, including yeast, bacteria or filamentous fungi. Examples of such software include Twist Codon Optimization tool software (supplied by Twist Biosciences), GenSmart^{™} Codon Optimization software (supplied by GenScript and whose functionality is described in application WO2020024917A1), IDT Codon Optimization Tool software (supplied by Integrated DNA technologies), and Azenta's codon optimization tool software (supplied by Azenta).

When the nucleic acid sequence is further optimized for expression in yeast, it is advantageously optimized for expression in yeast of the genus *Saccharomyces,* preferably of the species S. *cerevisiae.*

When the nucleic acid sequence is further optimized for expression in a bacterium, it is advantageously optimized for expression in a bacterium of the genera Escherichia (particularly strains of the species *Escherichia coli,* the species most widely used for recombinant protein production) and Pseudomonas.

According to one embodiment, the nucleic acid molecule is isolated from a heterologous vector or host cell comprising said molecule, said vector or said host cell being any vector or any host cell as defined above and as described below in the sections "Host cells" or "Vectors".

According to one embodiment, the nucleic acid molecule is synthesized *in vitro* by means of nucleic synthesis techniques that those skilled in the art know perfectly well how to define. According to one embodiment, the nucleic acid molecule is recombinant.

### Vector

The present invention relates to vectors comprising at least one nucleic acid molecule according to the invention, or expressing the mutant polypeptide according to the invention.

Advantageously, the nucleic acid molecule is any nucleic acid molecule as defined above in section "Nucleic acid molecule".

Advantageously, the mutant polypeptide is any mutant polypeptide as defined above in section "Mutant polypeptide".

The vectors that are suitable in the context of the present invention comprise, without limitation, bacteriophage, plasmid or cosmid vectors for expression in prokaryotic host cells such as bacteria (for example *E. coli,* or bacteria of the *Pseudomonas* genus); vectors for expression in yeast (for example *Saccharomyces cerevisiae, Schyzosaccharomyces pombe, Pichia pastoris*); baculovirus vectors for expression in insect cell systems (for example, Sf 9 cells); viral and plasmid vectors for expression in plant cell systems (for example, the Ti plasmid, the cauliflower mosaic virus CaMV, the tobacco mosaic virus TMV); and also viral and plasmid vectors for expression in higher eukaryotic cells or organisms.

These vectors are generally commercially available (for example, from suppliers such as Invitrogen, Stratagene, Amersham Biosciences, Promega, etc.), available from deposit institutions such as the American Type Culture Collection (ATCC, Rockville, Md.), or have been the subject of numerous publications describing their sequences, their structures and the methods for producing them, so that those skilled in the art can apply them without difficulty.

Advantageously, the vector is a plasmid.

Representative examples of suitable plasmid vectors, in particular for expression in yeast (such as *Saccharomyces* species, in particular *Saccharomyces cerevisiae*) comprise, without limitation, pREP4, pCEP4 (Invitrogen), pCI (Promega), pVAX (Invitrogen) and pgWiz (Gene Therapy System Inc).

### Host cell - Composition

In another aspect, the present invention relates to a host cell comprising or expressing the mutant polypeptide according to the invention, or comprising the nucleic acid molecule according to the invention, or comprising the vector according to the invention, or any combination thereof.

Advantageously, the mutant polypeptide is any mutant polypeptide as defined above in section "Mutant polypeptide".

Advantageously, the nucleic acid molecule is any nucleic acid molecule as defined above in section "Nucleic acid molecule".

Advantageously, the vector is any vector as defined above in section "Vector".

According to various embodiments, said host cell, in particular said heterologous host cell mentioned above, can be a prokaryotic cell, a lower eukaryotic cell such as a yeast cell, and other eukaryotic cells such as insect cells, plant cells and mammalian cells (for example human or non-human cells, preferably non-human cells). When the host cell is an embryonic cell or an embryonic stem cell, the cell is a non-human cell.

Advantageously, the host cell is a microorganism selected from bacteria, yeasts, fungi, algae and cyanobacteria, more preferably a microorganism selected from yeasts, fungi, algae and bacteria, even more preferably a microorganism selected from yeasts and bacteria.

The host cell is preferably a yeast, said yeast being in particular selected from the *Saccharomyces, Candida, Ashbya, Dekkera, Pichia (Hansenula), Debaryomyces, Clavispora, Lodderomyces, Yarrowia, Zigosaccharomyces, Schizosaccharomyces, Torulaspora, Kluyveromyces, Brettanomycces, Cryptococcus* and *Malassezia* genera.

Even more particularly, the yeast is selected from the species *Saccharomyces cerevisiae, Saccharomyces boulardii, Saccharomyces douglasii, Saccharomyces bayanus, Zigosaccharomyces bailii, Schizosaccharomyces pombe, Dekkera brucelensis, Dekkera intermedia, Brettanomycces custersii, Brettanomycces intermedius, Kluyveromyces themotolerens, Torulaspora globosa* and *Torulaspora glabrata.*

Even more particularly, the yeast is of the *Saccharomyces* genus, preferably of the species *Saccharomyces cerevisiae.*

The host cell may also be preferably a bacterium, said bacterium being in particular selected from the Proteobacteria, Actinomycetes and Firmicutes bacteria, more preferably said bacterium is selected from the *Escherichia, Pseudomonas, Streptomyces, Corynebacterium, Bacillus,* and *Lactobacillus* genera. Even more particularly, the bacterium is from the *Escherichia* genus, preferably of the species *Escherichia* coli.

According to one embodiment, the host cell comprises at least one copy of the nucleic acid molecule as defined above, integrated into its genome.

According to one embodiment, the host cell comprises a single copy of the nucleic acid molecule as defined above, integrated into its genome.

When the host cell is a yeast cell, the copy or copies of the nucleic acid molecule can be integrated at various loci, preferentially at the URA3 locus, at the JLP1 locus, at the LEU2 locus, or at the TRP1 locus of the genome of said yeast cell. When the host cell is a yeast cell and several copies of the nucleic acid molecule are integrated, the various copies can be integrated at the same locus, or else at different loci, preferentially at any one of the combinations of the URA3, JLP1, LEU2 and/or TRP1 loci.

Advantageously, the codons used in the nucleic acid molecule have been adapted for optimal expression in the host cell selected (see above section relating to nucleic acid molecules of the invention).

To this effect, those skilled in the art know which technique to use in order to modify the codons of the nucleic acid molecule. The codons can for example be modified by *in vitro* site-directed mutagenesis using a sample of the nucleic acid molecule of which the codons are to be adapted, by means of an amplification by polymerase chain reaction (PCR). Alternatively, the nucleic acid molecule can be synthesized *in vitro* directly with the optimized codons.

The host cells can be cultured/grown in small-scale and large-scale, aerobic or anaerobic bioreactors, in flasks or in Petri dishes. The culture can be performed at a temperature, at a pH, in a culture medium and at an oxygen content that are suitable for a given host cell.

The present invention also relates to a composition comprising, consisting essentially of, or consisting of, the mutant polypeptide according to the invention, the nucleic acid molecule according to the invention, the vector according to the invention, the host cell according to the invention, or any combination thereof.

Advantageously, the mutant polypeptide is any mutant polypeptide as defined above in section "Mutant polypeptide".

Advantageously, the nucleic acid molecule is any nucleic acid molecule as defined above in section "Nucleic acid molecule".

Advantageously, the vector is any vector as defined above in section "Vector". Advantageously, the host cell is any host cell as defined above.

### Methods and uses

### Method for producing a mutant polypeptide according to the invention

The present invention also relates to a method for producing a mutant polypeptide of the invention, preferably any mutant polypeptide with phloroglucinol synthase activity as defined above.

According to one embodiment, the method for producing a mutant polypeptide as defined above comprises at least the steps consisting of:
(i) introducing a nucleic acid molecule or a vector as described above into a suitable host cell in accordance with the preceding description; and
(ii) culturing, *in vitro,* said host cell obtained in step (i) under conditions which allow the growth of said host cell and/or the expression of said nucleic acid molecule, so as to produce said mutant polypeptide.

According to another embodiment, the method for producing a polypeptide with phloroglucinol synthase activity as defined above comprises at least the step of:
(i) culturing, *in vitro,* a host cell expressing said mutant polypeptide, for example a host cell as described above, under conditions which allow the growth of said host cell and/or the expression of the nucleic acid molecule contained in said host cell, so as to produce said mutant polypeptide.

According to one embodiment, the method for producing a mutant polypeptide according to the invention comprises at least one additional step selected from the steps of:
(α) recovering the cells expressing said mutant polypeptide, obtained after the culturing step; and
(β) purifying the mutant polypeptide from the cells recovered in step (α).

### Method/use for producing phloroglucinol

The invention further relates to a use of the mutant polypeptide according to the invention, the nucleic acid molecule according to the invention, the vector according to the invention, the host cell according to the invention, the composition according to the invention, or any combination thereof, for producing phloroglucinol.

Advantageously, the mutant polypeptide is any mutant polypeptide as defined above in section "Mutant polypeptide".

Advantageously, the nucleic acid molecule is any nucleic acid molecule as defined above in section "Nucleic acid molecule".

Advantageously, the vector is any vector as defined above in section "Vector".

Advantageously, the host cell and/or the composition is(are) any host cell or composition as defined above in section "Host cell - Composition".

The present invention also relates to a method for producing phloroglucinol.

According to one embodiment M1, the method for producing phloroglucinol comprises at least the steps consisting in:
(i) obtaining cells by implementing one of the methods described above;
(ii) contacting the cells obtained in step (i) with a suitable substrate;
(iii) incubating the mixture resulting from step (ii) under conditions suitable for producing phloroglucinol;
(iv) optionally, recovering the reaction medium comprising the phloroglucinol, obtained after step (iii); and
(v) optionally, purifying the phloroglucinol from the reaction medium of step (iv).

According to another embodiment M2, the method for producing phloroglucinol comprises the steps consisting in:
a) contacting a host cell expressing the mutant polypeptide as defined above, for example a host cell as defined above, with a suitable substrate;
b) culturing, *in vitro,* the host cell of step a) under conditions which allow the growth of said host cell and/or the expression of the nucleic acid molecule contained in said host cell, so as to produce phloroglucinol;
c) optionally, recovering the phloroglucinol-containing culture medium, obtained after step b); and
d) optionally, purifying the phloroglucinol from the culture medium of step c).

For the purposes of methods M1 and M2, the substrate is a carbon source. Advantageously, the carbon source is a pure carbon source or an industrial coproduct (such as molasses or green syrup, for example from the sugar industry). Preferably, the substrate in the pure carbon source or the industrial coproduct is a simple sugar, such as glucose (or dextrose), fructose, galactose, mannose, sucrose, lactose or maltose; a complex sugar, such as a monosaccharide, a disaccharide or trisaccharides, or else a polysaccharide such as starch; an alcohol, such as ethanol; an acid; a fatty acid and the ester derivative thereof; or a mixture of sugars, of alcohols, of acids and/or of fatty acids or the ester derivatives thereof.

Preferably, the substrate is glucose. Alternatively, the substrate is ethanol.

According to another embodiment M3, the method for producing phloroglucinol comprises at least the steps consisting of:
(i) contacting at least one polypeptide obtained in step (B) of the method as described above with malonyl-CoA (MLC);
(ii) incubating the mixture resulting from step (i) under conditions suitable for producing phloroglucinol;
(iii) optionally, recovering the reaction medium comprising the phloroglucinol, obtained after step (ii); and
(iv) optionally, purifying the phloroglucinol from the reaction medium of step (iii).

According to another embodiment M4, the method for producing phloroglucinol comprises at least the steps consisting in:
a) contacting the mutant polypeptide as defined above with malonyl-CoA (MLC);
b) incubating the mixture resulting from step a) under conditions suitable for producing phloroglucinol;
c) optionally, recovering the phloroglucinol-containing reaction medium, obtained after step b); and
d) optionally, purifying the phloroglucinol from the reaction medium of step (iii).

According to one preferred embodiment, the purification of the phloroglucinol is carried out using any technique known from the skilled person, including (but not limited to) liquid-liquid extraction or crystallisation.

The examples which follow aim to illustrate the present invention without any limitation. The enzymes respectively encoded by the PHLD gene of *Pseudomonas fluorescens* (Zha et al., 2006), and by the PKS1 gene of *Ectocarpus siliculosus* (Meslet-Cladière et al., 2013) are used therein as controls.

### Sequences

The amino acid sequences of the wild-type polypeptide and the mutant polypeptides cited herein are summarized in Table 4 below.

**Table 4: PHLD Sequences**

| SEQ ID NO: | Name - Mutation(s) | Sequence |
|---|---|---|
| SEQ ID NO: 1 | PHLD.Tpu | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCELA ESLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 2 | Consensus | MNTTEQQSVLPQQAWLGAPPAPPTSVAVIESLATX35SPTQTYGQ AEX46ADX49VAARFDDPRQX60ERIRRVYAKTRVTERHLAIDPLTPE FAEFSX91RX93DTVRERMDLFFEHAAPLAIETARRALGDNX123X124 TDIGX129LVFVTSTGFLAPGVDVAVIRALGLX154PQTSRVVINFMG CAAAMNALRX176X177TDYVRAQPDRKSLX191ICLELSSVNAVFSGD PNDVVISSLFADGCGAAVIGASEVGHPLPX236GX238IVVRDTFTHL LDGAEDGIVLGVNX262NGITCX268LX270X271SLPQYIVNGVAPVVD AVLX290RNGLGREX298VAHWAIHPGGPKIIESAAAALGLPDDASR |
| | | LSWDVLAEHGNX339LSVSLLFVLERLRAQVASDGADGAPSTGMA FSFAPGVTVEGFLFDVVTTGE |
| SEQ ID NO: 3 | M27 (A124P, A154P, S177A, S236P, E268Q, E271R, A298D) | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCQL ARSLPQYIVNGVAPVVDAVLDRNGLGREDVAHWAIHPGGPKIIES AAAALGLPDDASRLSWDVLAEHGNMLSVSLLFVLERLRAQVASD GADGAPSTGMAFSFAPGVTVEGFLFDVVTTGE |
| SEQ ID NO: 4 | M28 (A124P, A154P, S177A, S236P, E268T, E271K, A298D) | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCTLA KSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 5 | A124P, A154P, S177A, S236P, E268S, A270S, E271K, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCSLS KSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 6 | A124P, A154P, S177A, S236P, E268Q, E271K, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCQL AKSLPQYIVNGVAPVVDAVLDRNGLGREDVAHWAIHPGGPKIIES AAAALGLPDDASRLSWDVLAEHGNMLSVSLLFVLERLRAQVASD GADGAPSTGMAFSFAPGVTVEGFLFDVVTTGE |
| SEQ ID NO: 7 | A124P, A154P, S177A, S236P, E268S, E271R, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCSLA RSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 8 | E268Q, E271K | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCQLA KSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 9 | E268T, E271K | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCTLA KSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 10 | E268T, E271R | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCTLA RSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 11 | A124P, A154P, S177A, S236P, E268Q, A270S, E271K, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD |
| | | YVRAQPDRKSLMICLELSSVNAVFSGDPNDVVISSLFADGCGAAVI GASEVGHPLPPGQIVVRDTFTHLLDGAEDGIVLGVNANGITCQLS KSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 12 | A124P, A154P, S177A, S236P, E268T, A270S, E271R, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCTLS RSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 13 | A124P, A154P, S177A, S236P, E268T, A270S, E271V, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCTLS VSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 14 | A124P, A154P, S177A, S236P, E268S, E271K, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCSLA KSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 15 | Q129M, V176I | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGMLVFVTS TGFLAPGVDVAVIRALGLAPQTSRVVINFMGCAAAMNALRISTDY VRAQPDRKSLMICLELSSVNAVFSGDPNDVVISSLFADGCGAAVIG ASEVGHPLPSGQIVVRDTFTHLLDGAEDGIVLGVNANGITCELAE SLPQYIVNGVAPVVDAVLDRNGLGREAVAHWAIHPGGPKIIESAA |
| | | AALGLPDDASRLSWDVLAEHGNMLSVSLLFVLERLRAQVASDGA DGAPSTGMAFSFAPGVTVEGFLFDVVTTGE |
| SEQ ID NO: 16 | M29 (E268Q, E271R) | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCQLA RSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 17 | A124P, A154P, S177A, S236P, E268Q, A270S, E271R, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCQLS RSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 18 | A124P, A154P, S177A, S236P, E268S, A270S, E271R, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCSLS RSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 19 | E271K | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCELA KSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 20 | S35G, A60R, A91R, A123S, A124P, A154P, | MNTTEQQSVLPQQAWLGAPPAPPTSVAVIESLATGSPTQTYGQA ESADRVAARFDDPRQRERIRRVYAKTRVTERHLAIDPLTPEFAEFS |
| | S177A, S236P, A270S, A298D | RRPDTVRERMDLFFEHAAPLAIETARRALGDNSPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCELS ESLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 21 | E268S, E271K | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCSLA KSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 22 | P93C, A262C | MNTTEQQSVLPQQAWLGAPPAPPTSVAVIESLATSSPTQTYGQA ESADRVAARFDDPRQAERIRRVYAKTRVTERHLAIDPLTPEFAEFS ARCDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNCNGITCELA ESLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 23 | E268T, E271V | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCTLA VSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 24 | A124P, A154P, S177A, S236P, E268T, A270S, E271K, A298D | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAPTDIGQLVFVTS TGFLAPGVDVAVIRALGLPPQTSRVVINFMGCAAAMNALRVATD YVRAQPDRKSLMICLELSSVNAVFSGDPNDVVISSLFADGCGAAVI GASEVGHPLPPGQIVVRDTFTHLLDGAEDGIVLGVNANGITCTLS |
| | | KSLPQYIVNGVAPVVDAVLDRNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 25 | E271R | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCELA RSLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 26 | S35G, S46A, A60R, A91R, A123S, A124P, A154P, S177A, M191V, S236P, Q238K, A270S, D290A, A298D | MNTTEQQSVLPQQAWLGAPPAPPTSVAVIESLATGSPTQTYGQA EAADRVAARFDDPRQRERIRRVYAKTRVTERHLAIDPLTPEFAEFS RRPDTVRERMDLFFEHAAPLAIETARRALGDNSPTDIGQLVFVTS TGFLAPGVDVAVIRALGLPPQTSRVVINFMGCAAAMNALRVATD YVRAQPDRKSLVICLELSSVNAVFSGDPNDVVISSLFADGCGAAVI GASEVGHPLPPGKIVVRDTFTHLLDGAEDGIVLGVNANGITCELS ESLPQYIVNGVAPVVDAVLARNGLGREDVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 27 | R49A | MNTTEQQSVLPQQAWLGAPPAPPTSVAVIESLATSSPTQTYGQA ESADAVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCELA ESLPQYIVNGVAPVVDAVLDRNGLGREAVAHWAIHPGGPKIIESA AAALGLPDDASRLSWDVLAEHGNMLSVSLLFVLERLRAQVASDG ADGAPSTGMAFSFAPGVTVEGFLFDVVTTGE |
| SEQ ID NO: 28 | M3391 | MNTTEQQSVLPQQAWLGAPPAPPTSVAVIESLATSSPTQTYGQA ESADRVAARFDDPRQAERIRRVYAKTRVTERHLAIDPLTPEFAEFS ARPDTVRERMDLFFEHAAPLAIETARRALGDNAATDIGQLVFVTS TGFLAPGVDVAVIRALGLAPQTSRVVINFMGCAAAMNALRVSTD YVRAQPDRKSLMICLELSSVNAVFSGDPNDVVISSLFADGCGAAVI GASEVGHPLPSGQIVVRDTFTHLLDGAEDGIVLGVNANGITCELA ESLPQYIVNGVAPVVDAVLDRNGLGREAVAHWAIHPGGPKIIESA AAALGLPDDASRLSWDVLAEHGNILSVSLLFVLERLRAQVASDGA DGAPSTGMAFSFAPGVTVEGFLFDVVTTGE |

The nucleic acid sequences encoding the wild-type polypeptide and the mutant polypeptides cited herein are summarized in Table 5 below.

**Table 5: Nucleic acid sequences encoding PHLD**

| **SEQ ID NO:** | **Name - Mutation(s)** | **Sequence** |
|---|---|---|
| SEQ ID NO: 46 | PHLD.Tpu | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTGAATTAGCAGAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 47 | M27 (A124P, A154P, S177A, S236P, E268Q, E271R, A298D) | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA |
| | | GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTCAATTAGCAAGAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 48 | M28 (A124P, A154P, S177A, S236P, E268T, E271K, A298D) | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG |
| | | AAGATGGGATAGTGCTGGGCGTCAACGCAAATGGGATCACT TGTACTTTAGCAAAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 49 | A124P, A154P, S177A, S236P, E268S, A270S, E271K, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTTCATTATCAAAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 50 | A124P, A154P, S177A, S236P, E268Q, E271K, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTCAATTAGCAAAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 51 | A124P, A154P, S177A, S236P, E268S, E271R, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC |
| | | CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTTCATTAGCAAGAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 52 | E268Q, E271K | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTCAATTAGCAAAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA |
| | | GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 53 | E268T, E271K | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTACTTTAGCAAAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 54 | E268T, E271R | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC |
| | | CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTACTTTAGCAAGAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 55 | A124P, A154P, S177A, S236P, E268Q, A270S, E271K, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG |
| | | TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTCAATTATCAAAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 56 | A124P, A154P, S177A, S236P, E268T, A270S, E271R, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTACTTTATCAAGAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT |
| | | CCTTCCACTGGGATGGCATTTTCATTTGCACCCGGTGTGACT GTAGAAGGGTTCCTGTTTGACGTGGTAACTACAGGCGAGTA G |
| SEQ ID NO: 57 | A124P, A154P, S177A, S236P, E268T, A270S, E271V, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTACTTTATCAGTTAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 58 | A124P, A154P, S177A, S236P, E268S, E271K, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG |
| | | AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTTCATTAGCAAAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 59 | Q129M, V1761 | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTATGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAATTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC |
| | | TTGTGAATTAGCAGAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 60 | M29 (E268Q, E271R) | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTCAATTAGCAAGAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 61 | A124P, A154P, S177A, S236P, E268Q, A270S, E271R, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTCAATTATCAAGAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 62 | A124P, A154P, S177A, S236P, E268S, A270S, E271R, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC |
| | | CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTTCATTATCAAGAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 63 | E271K | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTGAATTAGCAAAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA |
| | | GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 64 | S35G, A60R, A91R, A123S, A124P, A154P, S177A, S236P, A270S, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACAGGTAGCCCCACTCAAACCTATGGCC AAGCTGAATCCGCTGATCGTGTTGCCGCGAGATTTGATGACC CTAGACAGAGAGAACGTATTAGAAGGGTTTACGCTAAAACTC GTGTTACGGAAAGGCATTTAGCCATTGATCCATTGACTCCTG AGTTTGCTGAATTTAGCAGAAGGCCTGATACAGTTAGGGAAA GAATGGATTTATTTTTTGAACACGCAGCACCCTTAGCTATAG AAACAGCAAGAAGAGCCCTAGGCGATAATTCACCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTGAATTATCAGAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 65 | E268S, E271K | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC |
| | | CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTTCATTAGCAAAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 66 | P93C, A262C | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGTGCGATACAGTTAGGGAA AGAATGGATTTATTTTTTGAACACGCAGCACCCTTAGCTATA GAAACAGCAAGAAGAGCCCTAGGCGATAATGCGGCCACTGA TATTGGTCAGCTAGTTTTTGTGACATCTACGGGCTTTCTTGC ACCTGGTGTAGATGTTGCCGTTATCAGAGCCTTAGGTCTAGC GCCTCAAACCTCCAGAGTTGTTATTAATTTCATGGGATGTGC CGCTGCAATGAATGCCTTGAGAGTTAGTACCGATTATGTGAG AGCACAACCTGATAGAAAGTCACTGATGATTTGCTTAGAATT GAGTTCCGTCAATGCCGTATTTAGCGGTGACCCTAACGATGT |
| | | TGTCATTAGTTCATTGTTCGCTGATGGTTGCGGTGCAGCTGT AATAGGTGCTTCCGAAGTCGGCCATCCATTGCCTTCTGGCCA GATCGTTGTAAGGGATACGTTTACTCATTTGTTAGATGGTGC GGAAGATGGGATAGTGCTGGGCGTCAACTGCAATGGGATCA CTTGTGAATTAGCAGAAAGCCTTCCACAGTACATTGTCAACG GGGTAGCTCCCGTAGTCGACGCAGTTTTGGACAGGAACGGG TTGGGGAGGGAGGCAGTTGCACATTGGGCTATCCATCCTGG AGGTCCTAAAATAATAGAATCAGCAGCAGCTGCTTTAGGTCT ACCCGATGATGCATCTCGTTTGTCATGGGATGTCTTAGCTGA GCATGGAAATATGTTGTCTGTATCTCTGCTGTTTGTTTTAGA GAGATTAAGGGCCCAAGTTGCGAGCGATGGTGCGGATGGTG CTCCTTCCACTGGGATGGCATTTTCATTTGCACCCGGTGTGA CTGTAGAAGGGTTCCTGTTTGACGTGGTAACTACAGGCGAG TAG |
| SEQ ID NO: 67 | E268T, E271V | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTACTTTAGCAGTTAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC |
| | | TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 68 | A124P, A154P, S177A, S236P, E268T, A270S, E271K, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGCCTACTGATA TTGGTCAGCTAGTTTTTGTGACATCTACGGGCTTTCTTGCAC CTGGTGTAGATGTTGCCGTTATCAGAGCCTTAGGTCTACCTC CTCAAACCTCCAGAGTTGTTATTAATTTCATGGGATGTGCCG CTGCAATGAATGCCTTGAGAGTTGCAACCGATTATGTGAGAG CACAACCTGATAGAAAGTCACTGATGATTTGCTTAGAATTGA GTTCCGTCAATGCCGTATTTAGCGGTGACCCTAACGATGTTG TCATTAGTTCATTGTTCGCTGATGGTTGCGGTGCAGCTGTAA TAGGTGCTTCCGAAGTCGGCCATCCATTGCCTCCTGGCCAGA TCGTTGTAAGGGATACGTTTACTCATTTGTTAGATGGTGCGG AAGATGGGATAGTGCTGGGCGTCAACGCAAATGGGATCACT TGTACTTTATCAAAAAGCCTTCCACAGTACATTGTCAACGGG GTAGCTCCCGTAGTCGACGCAGTTTTGGACAGGAACGGGTT GGGGAGGGAGGATGTTGCACATTGGGCTATCCATCCTGGAG GTCCTAAAATAATAGAATCAGCAGCAGCTGCTTTAGGTCTAC CCGATGATGCATCTCGTTTGTCATGGGATGTCTTAGCTGAGC ATGGAAATATGTTGTCTGTATCTCTGCTGTTTGTTTTAGAGA GATTAAGGGCCCAAGTTGCGAGCGATGGTGCGGATGGTGCT CCTTCCACTGGGATGGCATTTTCATTTGCACCCGGTGTGACT GTAGAAGGGTTCCTGTTTGACGTGGTAACTACAGGCGAGTA G |
| SEQ ID NO: 69 | E271R | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG |
| | | AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTGAATTAGCAAGAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 70 | S35G, S46A, A60R, A91R, A123S, A124P, A154P, S177A, M191V, S236P, Q238K, A270S, D290A, A298D | ATGAATACCACTGAACAACAATCAGTCTTGCCTCAACAAGCTT GGCTGGGAGCACCACCAGCCCCTCCAACTAGCGTTGCAGTCA TAGAATCACTGGCTACAGGTAGCCCCACTCAAACCTATGGCC AAGCTGAAGCAGCTGATCGTGTTGCCGCGAGATTTGATGAC CCTAGACAGAGAGAACGTATTAGAAGGGTTTACGCTAAAACT CGTGTTACGGAAAGGCATTTAGCCATTGATCCATTGACTCCT GAGTTTGCTGAATTTAGCAGAAGGCCTGATACAGTTAGGGA AAGAATGGATTTATTTTTTGAACACGCAGCACCCTTAGCTAT AGAAACAGCAAGAAGAGCCCTAGGCGATAATTCACCTACTGA TATTGGTCAGCTAGTTTTTGTGACATCTACGGGCTTTCTTGC ACCTGGTGTAGATGTTGCCGTTATCAGAGCCTTAGGTCTACC TCCTCAAACCTCCAGAGTTGTTATTAATTTCATGGGATGTGC CGCTGCAATGAATGCCTTGAGAGTTGCAACCGATTATGTGAG AGCACAACCTGATAGAAAGTCACTGGTTATTTGCTTAGAATT GAGTTCCGTCAATGCCGTATTTAGCGGTGACCCTAACGATGT TGTCATTAGTTCATTGTTCGCTGATGGTTGCGGTGCAGCTGT AATAGGTGCTTCCGAAGTCGGCCATCCATTGCCTCCTGGCAA AATCGTTGTAAGGGATACGTTTACTCATTTGTTAGATGGTGC GGAAGATGGGATAGTGCTGGGCGTCAACGCAAATGGGATCA |
| | | CTTGTGAATTATCAGAAAGCCTTCCACAGTACATTGTCAACG GGGTAGCTCCCGTAGTCGACGCAGTTTTGGCAAGGAACGGG TTGGGGAGGGAGGATGTTGCACATTGGGCTATCCATCCTGG AGGTCCTAAAATAATAGAATCAGCAGCAGCTGCTTTAGGTCT ACCCGATGATGCATCTCGTTTGTCATGGGATGTCTTAGCTGA GCATGGAAATATGTTGTCTGTATCTCTGCTGTTTGTTTTAGA GAGATTAAGGGCCCAAGTTGCGAGCGATGGTGCGGATGGTG CTCCTTCCACTGGGATGGCATTTTCATTTGCACCCGGTGTGA CTGTAGAAGGGTTCCTGTTTGACGTGGTAACTACAGGCGAG TAG |
| SEQ ID NO: 71 | R49A | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATGCAGTTGCCGCAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTGAATTAGCAGAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATGTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |
| SEQ ID NO: 72 | M3391 | ATGAATACCACTGAACAACAATCAGTCTTGCCTCAACAAGCTT GGCTGGGAGCACCACCAGCCCCTCCAACTAGCGTTGCAGTCA TAGAATCACTGGCTACATCAAGCCCCACTCAAACCTATGGCC AAGCTGAATCCGCTGATCGTGTTGCCGCGAGATTTGATGACC CTAGACAGGCTGAACGTATTAGAAGGGTTTACGCTAAAACTC GTGTTACGGAAAGGCATTTAGCCATTGATCCATTGACTCCTG AGTTTGCTGAATTTAGCGCAAGGCCTGATACAGTTAGGGAAA GAATGGATTTATTTTTTGAACACGCAGCACCCTTAGCTATAG AAACAGCAAGAAGAGCCCTAGGCGATAATGCGGCCACTGATA TTGGTCAGCTAGTTTTTGTGACATCTACGGGCTTTCTTGCAC CTGGTGTAGATGTTGCCGTTATCAGAGCCTTAGGTCTAGCG CCTCAAACCTCCAGAGTTGTTATTAATTTCATGGGATGTGCC GCTGCAATGAATGCCTTGAGAGTTAGTACCGATTATGTGAGA GCACAACCTGATAGAAAGTCACTGATGATTTGCTTAGAATTG AGTTCCGTCAATGCCGTATTTAGCGGTGACCCTAACGATGTT GTCATTAGTTCATTGTTCGCTGATGGTTGCGGTGCAGCTGTA ATAGGTGCTTCCGAAGTCGGCCATCCATTGCCTTCTGGCCAG ATCGTTGTAAGGGATACGTTTACTCATTTGTTAGATGGTGCG GAAGATGGGATAGTGCTGGGCGTCAACGCAAATGGGATCAC TTGTGAATTAGCAGAAAGCCTTCCACAGTACATTGTCAACGG GGTAGCTCCCGTAGTCGACGCAGTTTTGGACAGGAACGGGT TGGGGAGGGAGGCAGTTGCACATTGGGCTATCCATCCTGGA GGTCCTAAAATAATAGAATCAGCAGCAGCTGCTTTAGGTCTA CCCGATGATGCATCTCGTTTGTCATGGGATGTCTTAGCTGAG CATGGAAATATTTTGTCTGTATCTCTGCTGTTTGTTTTAGAG AGATTAAGGGCCCAAGTTGCGAGCGATGGTGCGGATGGTGC TCCTTCCACTGGGATGGCATTTTCATTTGCACCCGGTGTGAC TGTAGAAGGGTTCCTGTTTGACGTGGTAACTACAGGCGAGT AG |

The sequences cited herein other than that the wild-type polypeptide and the mutant polypeptides are summarized in **Table 6** below.

**Table 6: Other Sequences**

| **SEQ ID NO:** | **Name** | **Sequence** |
|---|---|---|
| SEQ ID NO: 29 | S. *cerevisiae* ADH2 promoter | TATCTTAACTGATAGTTTGATCAAAGGGGCAAAACGTAGGGGCAAACA AACGGAAAAATCGTTTCTCAAATTTTCTGATGCCAAGAACTCTAACCA GTCTTATCTAAAAATTGCCTTATGATCCGTCTCTCCGGTTACAGCCTG |
| | | TGTAACTGATTAATCCTGCCTTTCTAATCACCATTCTAATGTTTTAATT AAGGGATTTTGTCTTCATTAACGGCTTTCGCTCATAAAAATGTTATGA CGTTTTGCCCGCAGGCGGGAAACCATCCACTTCACGAGACTGATCTCC TCTGCCGGAACACCGGGCATCTCCAACTTATAAGTTGGAGAAATAAGA GAATTTCAGATTGAGAGAATGAAAAAAAAAAAAAAAAAAGGCAGAGGA GAGCATAGAAATGGGGTTCACTTTTTGGTAAAGCTATAGCATGCCTAT CACATATAAATAGAGTGCCAGTAGCGACTTTTTTCACACTCGAAATAC TCTTACTACTGCTCTCTTGTTGTTTTTATCACTTCTTGTTTCTTCTTGG TAAATAGAATATCAAGCTACAAAAAGCATACAATCAACTATCAACTATT AACTATATCGTAATACACA |
| SEQ ID NO: 30 | *S. cerevisiae* CCW12 promoter | AACCAGGGCAAAGCAAAATAAAAGAAACTTAATACGTTATGCCGTAAT GAAGGGCTACCAAAAACGATAATCTCAACTGTAAACAGGTACAATGCG GACCCTTTTGCCACAAAACATACATCATTCATTGCCGGAAAAAGAAAG AAGTGAAGACAGCAGTGCAGCCAGCCATGTTGCGCCAATCTAATTATA GATGCTGGTGCCCTGAGGATGTATCTGGAGCCAGCCATGGCATCATG CGCTACCGCCGGATGTAAAATCCGACACGCAAAAGAAAACCTTCGAGG TTGCGCACTTCGCCCACCCATGAACCACACGGTTAGTCCAAAAGGGGC AGTTCAGATTCCAGATGCGGGAATTAGCTTGCTGCCACCCTCACCTCA CTAACGCTGCGGTGTGCGGATACTTCATGCTATTTATAGACGCGCGT GTCGGAATCAGCACGCGCAAGAACCAAATGGGAAAATCGGAATGGGT CCAGAACTGCTTTGAGTGCTGGCTATTGGCGTCTGATTTCCGTTTTG GGAATCCTTTGCCGCGCGCCCCTCTCAAAACTCCGCACAAGTCCCAGA AAGCGGGAAAGAAATAAAACGCCACCAAAAAAAAAAATAAAAGCCAAT CCTCGAAGCGTGGGTGGTAGGCCCTGGATTATCCCGTACAAGTATTT CTCAGGAGTAAAAAAACCGTTTGTTTTGGAATTCCCCATTTCGCGGCC ACCTACGCCGCTATCTTTGCAACAACTATCTGCGATAACTCAGCAAAT TTTGCATATTCGTGTTGCAGTATTGCGATAATGGGAGTCTTACTTCCA ACATAACGGCAGAAAGAAATGTGAGAAAATTTTGCATCCTTTGCCTCC GTTCAAGTATATAAAGTCGGCATGCTTGATAATCTTTCTTTCCATCCT ACATTGTTCTAATTATTCTTATTCTCCTTTATTCTTTCCTAACATACCA AGAAATTAATCTTCTGTCATTCGCTTAAACACTATATCAATA |
| SEQ ID NO: 31 | *S. cerevisiae* TEF1 promoter | CATAGCTTCAAAATGTTTCTACTCCTTTTTTACTCTTCCAGATTTTCTC GGACTCCGCGCATCGCCGTACCACTTCAAAACACCCAAGCACAGCATA CTAAATTTCCCCTCTTTCTTCCTCTAGGGTGTCGTTAATTACCCGTACT AAAGGTTTGGAAAAGAAAAAAGAGACCGCCTCGTTTCTTTTTCTTCGT CGAAAAAGGCAATAAAAATTTTTATCACGTTTCTTTTTCTTGAAAATTT TTTTTTTTGATTTTTTTCTCTTTCGATGACCTCCCATTGATATTTAAGT TAATAAACGGTCTTCAATTTCTCAAGTTTCAGTTTCATTTTTCTTGTTC TATTACAACTTTTTTTACTTCTTGCTCATTAGAAAGAAAGCATAGCAAT CTAATCTAAG |
| SEQ ID NO :73 | *S. cerevisiae* PGK1 promoter (pPGK1) | ACTCTTTTCTTCTAACCAAGGGGGTGGTTTAGTTTAGTAGAACCTCGT GAAACTTACATTTACATATATATAAACTTGCATAAATTGGTCAATGCAA GAAATACATATTTGGTCTTTTCTAATTCGTAGTTTTTCAAGTTCTTAGA TGCTTTCTTTTTCTCTTTTTTACAGATCATCAAGGAAGTAATTATCTAC TTTTTACAACAAATATAAAACAA |
| SEQ ID NO :74 | *S. cerevisiae* TDH3 promoter (pTDH3) | AGTTTATCATTATCAATACTGCCATTTCAAAGAATACGTAAATAATTAA TAGTAGTGATTTTCCTAACTTTATTTAGTCAAAAAATTAGCCTTTTAAT TCTGCTGTAACCCGTACATGCCCAAAATAGGGGGCGGGTTACACAGA ATATATAACATCGTAGGTGTCTGGGTGAACAGTTTATTCCTGGCATCC ACTAAATATAATGGAGCCCGCTTTTTAAGCTGGCATCCAGAAAAAAAA AGAATCCCAGCACCAAAATATTGTTTTCTTCACCAACCATCAGTTCATA GGTCCATTCTCTTAGCGCAACTACAGAGAACAGGGGCACAAACAGGC AAAAAACGGGCACAACCTCAATGGAGTGATGCAACCTGCCTGGAGTA AATGATGACACAAGGCAATTGACCCACGCATGTATCTATCTCATTTTC TTACACCTTCTATTACCTTCTGCTCTCTCTGATTTGGAAAAAGCTGAAA AAAAAGGTTGAAACCAGTTCCCTGAAATTATTCCCCTACTTGACTAAT AAGTATATAAAGACGGTAGGTATTGATTGTAATTCTGTAAATCTATTT CTTAAACTTCTTAAATTCTACTTTTATAGTTAGTCTTTTTTTTAGTTTT AAAACACCAAGAACTTAGTTTCGAATAAACACACATAAACAAACAAA |
| SEQ ID NO :75 | *S. cerevisiae* TP11 promoter (pTPI1) | CTACTTATTCCCTTCGAGATTATATCTAGGAACCCATCAGGTTGGTGG AAGATTACCCGTTCTAAGACTTTTCAGCTTCCTCTATTGATGTTACAC CTGGACACCCCTTTTCTGGCATCCAGTTTTTAATCTTCAGTGGCATGT GAGATTCTCCGAAATTAATTAAAGCAATCACACAATTCTCTCGGATAC CACCTCGGTTGAAACTGACAGGTGGTTTGTTACGCATGCTAATGCAAA GGAGCCTATATACCTTTGGCTCGGCTGCTGTAACAGGGAATATAAAG GGCAGCATAATTTAGGAGTTTAGTGAACTTGCAACATTTACTATTTTC CCTTCTTACGTAAATATTTTTCTTTTTAATTCTAAATCAATCTTTTTCA ATTTTTTGTTTGTATTCTTTTCTTGCTTAAATCTATAACTACAAAAAAC ACATACATAAACTAAAA |
| SEQ ID NO: 32 | *S*. *cerevisiae* RPL3 terminator | GAAGTTTTGTTAGAAAATAAATCATTTTTTAATTGAGCATTCTTATTCC TATTTTATTTAAATAGTTTTATGTATTGTTAGCTACATACAACAGTTTA AATCAAATTTTCTTTTTCCCAAGTCCAAAATGGAGGTTTATTTTGATG ACCCGCATGCGATTATGTTTTGAAAGTATAAGACTACATACATGTACA TATATTTAAACATGTAAACCCGTCCATTATATTGCTTACTTTCTTCTTT TTTGCCGTTTTGACTTGGACCTCTGGTTTGCTATTTCCTTACAATCTT TGCTACAAT |
| SEQ ID NO: 33 | *S*. *cerevisiae* ADH1 terminator | GCGAATTTCTTATGATTTATGATTTTTATTATTAAATAAGTTATAAAAA AAATAAGTGTATACAAATTTTAAAGTGACTCTTAGGTTTTAAAACGAA AATTCTTATTCTTGAGTAACTCTTTCCTGTAGGTCAGGTTGCTTTCTC AGGTATAGCATGAGGTCGCTCTTATTGACCACACCTCTACCGG |
| SEQ ID NO :76 | *S*. *cerevisiae* CYC1 terminator | GCCCGGTACCAAGATGGCCTTTGGTGGGTTGAAGAAGGAAAAAGACA GAAACGACTTAATTACCTACTTGAAAAAAGCCTGTGAGTAAACAGGCC CCTTTTCCTTTGTCGATATCATGTAATTAGTTATGTCACGCTTACATTC ACGCCCTCCCCCCACATCCGCTCTAACCGAAAAGGAAGGAGTTAGACA ACCTGAAGTCTAGGTCCCTATTTATTTTTTTATAGTTATGTTAGTATTA AGAACGTTATTTATATTTCAAATTTTTCTTTTTTTTCTGTACAGACGCG TGTACGCATGTAACATTATACTGAAAACCTTGCTTGAGAAG |
| SEQ ID NO :34 | FLAG peptide | DYKDDDK |
| SEQ ID NO: 35 | Strep-tag | WSHPQFEK |
| SEQ ID NO: 36 | Strep-tag | AWAHPQPGG |
| SEQ ID NO : 37 | B-tag | QYPALT |
| SEQ ID NO : 38 | EE-tag | EYMPME |
| SEQ ID NO : 39 | EE-tag | EFMPME |
| SEQ ID NO : 40 | T7-tag | MASMTGGQQMG |
| SEQ ID NO : 41 | V5-tag | GKPIPNPLLGLDST |
| SEQ ID NO : 42 | E2 | SSTSSDFRDR |
| SEQ ID NO : 43 | HAT | KDHLIHNVHKEFHAHAHNK |
| SEQ ID NO : 44 | AU1 | DTYRYI |
| SEQ ID NO : 45 | AU5 | TDFYLK |

### DESCRIPTION OF THE FIGURES

**Figure 1** presents the homology model of PhlD_Tpu in homodimeric form. The two chains are represented in black and grey, respectively.
**Figure 2** shows the map of YCplac22_PGK CYC TEF ADHCYC plasmid (low copy).
**Figure 3** shows a PCR product of site directed mutagenesis.
**Figure 4** shows the Map of pET-26b-PhlD-Tpu.
**Figure 5** is a graph showing 100µM malonyl-CoA (MLC) (in black) and CoA (in grey) standard solutions separated using HPLC.

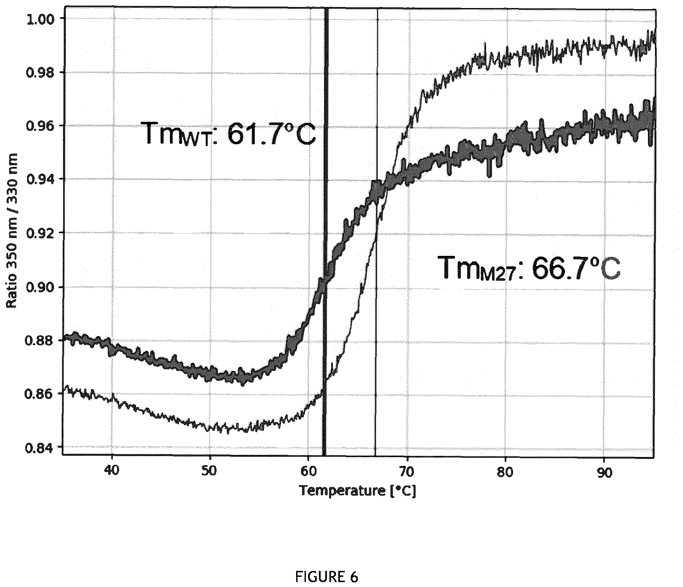

**Figure 6** shows the Tm determination of purified PhlD_Tpu M27 (Grey) and PhlD_Tpu WT (Black) by nanoDSF.

### EXAMPLES

### 1. EXAMPLE 1: Design of a novel and comprehensive strategy for producing optimized phloroglucinol synthases - Materials & Methods

### 1.1. Molecular modelling approaches

### 1.1.1. Modelling of PhlD_Tpu and modelling of PhlD_Tpu:ligand complexes

A 3D model of the Phld_Tpu homodimer was constructed using a combination of comparative modelling. The crystal structure of the type III polyketide synthase PKS18 from *Mycobacterium tuberculosis* was used as template. To procure a reasonable model, ~1,500 structures were calculated. The lowest energy modelled structure was selected to represent a fully folded and stable conformation. The backbone RMSD between the final modelled structure and the template structures is 0.9Å.

Next, we modelled PhlD_Tpu in complex with Malonyl-CoA (MLC) substrate. The docking site for MLC was decided based on structural analysis of other available PKS III structures co-crystalized with MLC (for example PDB code: 1EE0) from the PDB database.

Two putative transient binding modes of Phlo were modelled based on literature data. The first binding mode was identified based on the product binding site located at the dimer interface reported in crystallographic structures of other PKS structures (PDB code: 4B0N, PKS-I from *E*. *siliculous* with malonic acid as product, Meslet-Cladière et al., 2013). In that case, protein docking was simultaneously applied to generate a symmetric complex structure starting from the MLC docked monomer. An alternative binding mode was generated with the Phlo located close to the catalytic triad (C167, H305, N338) in absence of MLC.

### 1.1.2 Analysis of models and simulations

Molecular dynamics simulations were performed for all systems in presence of explicit solvent.

Molecular dynamics simulations were further analysed using cross-correlation of atomic fluctuations (Lange O. et al., 2006) in order to understand coupled motions of different residues from molecular dynamics simulation trajectories and identify potential allosteric communication pathways.

Simulations were also analysed to identify residues involved in substrate and product binding. The per residue energy binding contribution for substrate (MLC) and product (Phlo) was calculated using the MM-GBSA method. The python script, MMGBSA.py (Miller B. et al., 2012) from the AmberTools package was used to perform the calculations. For each set of PhlD_Tpu complexed systems, the initial 5 nanoseconds (ns) of each trajectory (out of two independent simulations) was concatenated resulting in a total of 10 ns trajectory, which was then used for binding energy calculations.

The PROSS design method (Goldenzweig A. et al., 2016) was applied to identify the design positions. For that, we used the multiple sequence alignment of enzymes in the PKSIII family to find variable regions. Highly conserved regions were not included as they are involved in protein folding and function. Additionally, regions that were close to ligand binding and protein-protein interface were also excluded (residues 1-5, 8, 23, 37, 43, 57-59, 62-63, 65-66, 68-69, 70-72, 75, 93-97, 99-100, 103-104, 107-108, 110, 128-130, 132, 134-149, 155-169, 172, 175-176, 179-180, 182-187, 194-195, 205-208, 210-218, 222, 245-251, 253-269, 271-277, 284, 291, 302, 304-314, 330, 333, 337-338, 340-341, 350, 358, 364, 366, 371-377, 379, 386-388). Once the positions were identified on the protein surface, computational deep screening was performed to identify the nature/type of mutations (type of residue mutation) at each position which were predicted to improve enzyme thermostability compared to the wild type. The nature/type of mutation to include at each position for deep screening was decided based on the residue variability at that position in the multiple sequence alignment of the PKSIII family. On this basis, we designed a library of PhlD_Tpu mutants.

To investigate the release of Phlo from the active site, we used Random Accelerated Molecular Dynamics simulations to compute the Phlo release pathway. Amino acid in contact with the Phlo during its release were identified.

Molecular dynamics simulations of the PhlD_Tpu dimer were analyzed, leading to the identification of 34 residues that were further targeted by mutagenesis. Some residues were also identified to introduce disulphide bridges.

Finally, we used ancestral sequence reconstruction (ASR), shown to be a useful approach for engineering proteins with enhanced properties such as improved activity and/or stability. Here, we used the ASR approach to reconstruct the PhlD ancestral sequences of the Actinobacteria phylum subtree.

PhlD_Tpu homolog sequences belonging to the Actinobacteria phylum were retrieved from the Uniprot database (Uniprot database release 2022_02) using the EFI-EST (Zallot, R. et al., 2019). In addition to the 2369 sequences obtained with EFI-EST, four sequences previously identified from the natural diversity and nine sequences described in patent WO2019002799A1 were added to the sequence set. Next, the redundant sequences were removed with the CD-Hit software using a sequence identity threshold of 90%. Finally, sequences with less than 40 % identity with Phld_Tpu were removed resulting in a final set of 52 sequences. Following the same protocol, five homolog sequences were selected from the Cyanobacteria phylum and were used as outgroup for the phylogenic tree rooting (described below).

The 57 sequences were subsequently aligned using MAFFT (Katoh K. et al., 2002) and N- and C-termini were trimmed as they showed low sequence conservation. The resulting Multiple Sequence Alignment (MSA) was used to construct the phylogenic tree with the iq-tree software (Minh, B. et al., 2020). A total of 168 substitution models were tested and the best model was selected based on the Bayesian Information Criterion (LG+F+I+G4) and used for the phylogenic tree construction. The unrooted tree generated was finally rooted by selecting the Cyanobacteria sequences as outgroup.

The phylogenic tree was used to reconstruct the ancestral sequences corresponding to each tree node with GRASP (Foley G. et al., 2022). Sequences were inferred using the marginal reconstruction approach which gives the probability distribution of each residue for a single ancestor. Sequences corresponding to the three closest nodes (ancestors) to PhlD_Tpu were analyzed. Mutation located at the N-terminal or with a low posterior probability (p<0.7) were reverted to the wild type amino acids.

### 1.2. Experimental methods

### 1.2.1. Site directed mutagenesis method

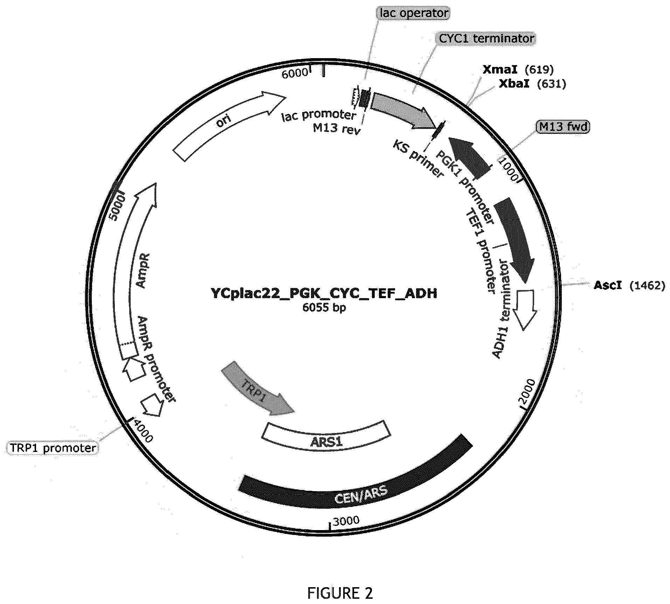

Site directed mutagenesis was employed to produce single mutants for the selected target sites, using standard method. Briefly, inverse PCR, which utilizes non-overlapping primers with one containing the intended mutation, was used to introduce point mutations resulting in the amplification of a linear, double-stranded, mutated product. For each target residue, a forward primer containing the desired amino acid substitution was used in a PCR reaction containing ClonAMP HiFi PCR Premix (a pre-assembled mix of enzyme, optimized buffer, and dNTPs) enabling quick and easy setup of the PCR reaction. The template used for mutagenesis was the PhlD_Tpu (wild type (WT)) which was cloned in YCplac22_PGK CYC TEF ADH yeast expression vector (low copy) under the control of pTEF1 promoter and with ADH terminator as shown in Figure 2. It is a shuttle vector that contains two origins of replication to promote its propagation in yeast and *E. coli.* Trp1 gene serves as an auxotrophic marker for selection in TRP- yeast, whereas an ampicillin resistance gene allows for selection in *E. coli.*

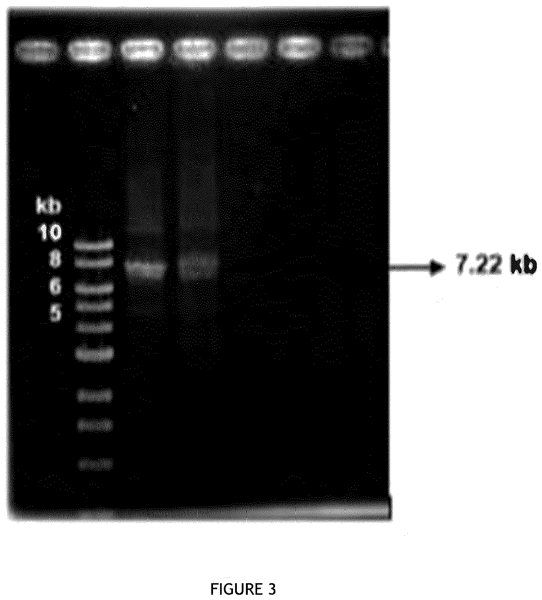

It is important to note that the PhlD_Tpu mutants were expressed using the YCplac22_PGK CYC TEF ADH vector, which did not include the ACC1 gene. This implies that these mutants solely depend on the malonyl-CoA produced by ACC1 encoded by the ACC gene present in the yeast genome only. The PCR product visualized by 0.8% agarose gel electrophoresis reveals the presence of a band at 7.2 kb (5 kb= vector + 2 kb insert; **Figure 3****,** kb=kilobase).

Following PCR amplification, the mutagenic plasmids were digested using the methylation sensitive endonuclease *Dpn*I by incubating at 37°C for 1 hour or overnight, subjected to treatment with polynucleotide kinase (T4 PNK) to add a phosphate group, and ligated. The resulting ligated product was transformed into *E. coli* TOP 10 cells and positive clones were selected after overnight incubation at 37°C. Plasmids were then extracted from the positive clones using the QIAprep^{®} Spin Miniprep Kit (Qiagen) and sequenced via Sanger sequencing (Eurofins). The confirmed PhlD_Tpu variants were transformed into competent cell of *Saccharomyces cerevisiae,* as prepared by the protocol of Gietz et al., 2014. (LiAc/SS carrier DNA/PEG method).

### 1.2.2. Generation of multi-position yeast mutant library

The custom library was designed for PhlD_Tpu with eight target positions and the amino acids that need to be substituted at each position are defined rationally, derived from the experimental knowledge of site-directed mutagenesis. The custom designed library was obtained from commercial provider GenScript. The target gene PhlD_Tpu cloned in plasmid-YCplac22_PGK CYC TEF ADH was sent to GenScript and the custom designed combinatorial library was prepared with their indigenous technology. The library was delivered in clonable plasmid and directly used for transformation into *Saccharomyces cerevisiae, which* was made competent by the LiAc/SS carrier DNA/PEG method, followed by high throughput screening.

### 1.2.3. Phloroglucinol production assay in yeast mutants

After transformation, clones were selected based on yeast auxotrophy to tryptophan. Phlo production was evaluated in two different scales; low- & high- throughput scales. For Site Saturation Mutagenesis (SSM) library, the low throughput scale was carried out manually where the transformed clones were picked, inoculated into 96 deep well plates (DWP) and incubated at 30° C for 24 hours growth. With multichannel pipettes, 10 µL of grown culture were transferred in fresh 500 µL of Trp_drop out CS media (yeast nitrogen base without amino acid and without ammonium sulfate 1.7 g/L, ammonium chloride 2.03 g/L, complete synthetic drop put mixture -Trp Formedium 0.74 g/L and glucose 2%) in 96 deep-well plates (3 DWP for three days sampling) and Phlo production was monitored at different time points (day 1, day 2 and day 3) with colorimetric reagent as described below.

On the other hand for custom library, Phlo production was performed in high throughput scale. For that, transformed colonies grown on QTrays, were picked (QPix2XT, Molecular Devices) and inoculated in standard 96-well plate, pre-filled with 200 µL of CS medium through liquid handling robot (Nimbus, Hamilton). After incubation at 30°C, 10 µL of overnight grown culture were transferred in fresh 500 µL of Trp_drop out CS media in 96 deep-well plates with automated liquid handling system. Protein induction was not necessary because the promoter used for expression is constitutive (pTEF1 promoter). The OD 600 nm density of cells was measured for all cultures at day 3 in microtitre plates prior to Phlo measurement. After centrifugation at 3700 rpm for 10 minutes, the production of Phlo present in the supernatant was measured. A colorimetric reagent, 4-Hydroxy-3-methoxycinnamladehyde (CAS: 458-36-6), at 500 mg/L in a HCl (37% (w/w))/ethanol solution (v:v 1:3) 150 µL, was added to 100 µL of supernatant, in parallel to the preparation of a standard curve of different Phlo concentrations ranging from 5 mg/L to 125 mg/L. This solution mixture was incubated at room temperature for 30-40 minutes before absorbance measurement with a microplate reader at 540 nm. Results from day 1 and 2 were excluded, as Phlo production is difficult to measure during this time, and only the results from day 3 were considered.

### 1.2.4. Subcloning into E. coli expression plasmid and overexpression

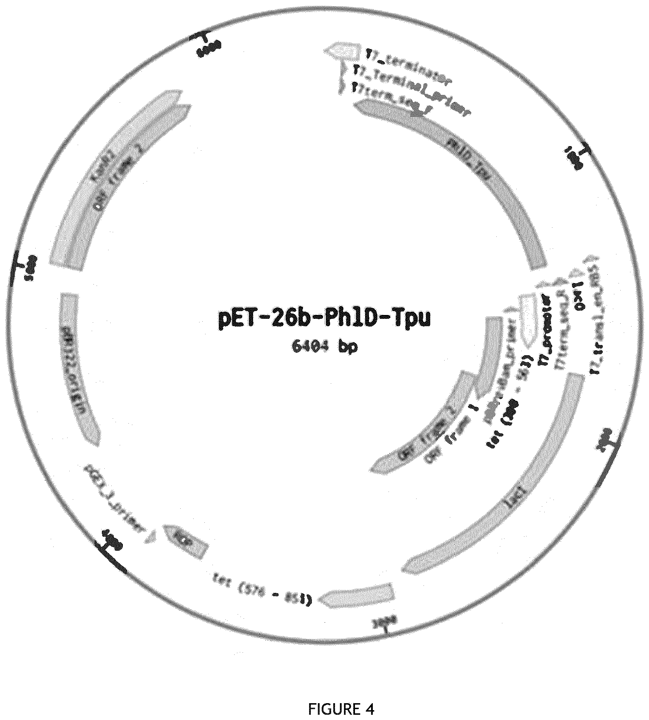

The selected PhlD_Tpu sequences are amplified from the yeast expression plasmid and fused with a 6xHis tag encoded at the C-terminus. This fusion is inserted into the pET26b(+) plasmid, between the *Xho*I and *Nde*I restriction sites (as shown in **Figure 4**)**,** with the *Lac*I promoter controlling its expression. The recombinant plasmid is used to transform *E. coli* BL21(DE3) competent cells, which are grown in LB medium (10 g/L NaCl, 10 g/L Tryptone, 5 g/L yeast extract) at 37° C with shaking, in a final volume of 500 mL. The expression of the recombinant plasmid is initiated by adding 0.1 mM of IPTG when the OD600nm reaches 0.4-0.5. The cells are then allowed to grow overnight at 20°C until they reach an OD600nm of approximately 1.5-1.6.

### 1.2.5. Purification

*E. coli* BL21(DE3) cells transformed as described in paragraph 1.2.4 above were disrupted by Fast-Prep at a speed setting of 6 m/sec for 3 cycles of 45 sec. The supernatant is filtered on a 0.45 µm membrane before being applied to a 1 mL Talon^{®} Resin column. The column is washed first with 10 column volumes of sodium phosphate buffer (50 mM, pH7; NaCl 100 mM) and with 10 column volumes of sodium phosphate buffer with 10 mM of imidazole (3% buffer B, Phosphate buffer 50 mM, 500 mM imidazole pH 8.5; 100mM NaCl). Protein elution is performed using 150 mM imidazole in sodium phosphate buffer (50 mM pH 7, NaCl 100mM). Collected fractions are pooled and diafiltrated using 5 volumes of sodium phosphate buffer (50 mM pH 7, NaCl 100mM) using a 30 kDa diafiltration unit (Amicon ^{®}Ultra, Merck) before reducing the volume to 500 µL.

The concentrate is applied on a gel filtration column (S200 TRICORN 10/300 Superdex 200). Elution is performed with an isocratic gradient of sodium phosphate buffer (50 mM pH 7, NaCl 100mM). The purity of the various fractions obtained is checked by SDS-PAGE (Mini-PROTEAN TGX Stain-Free Gels, Bio Rad)

### 1.2.6. Kinetic parameters determination

In order to access the kinetic parameters of PhlD_Tpu, the concentrations of MLC consumed and CoA produced were monitored by HPLC. The experiments were run for 120 sec and only the linear part of CoA variation with time was used to calculate initial velocity. The experiments were carried using a range of MLC concentrations (5-100 µM) in sodium phosphate buffer (50 mM pH 7), at 30°C, and with 0.5 µM of pure enzyme. At 10, 20, 30, 50, 60 and 120 sec, 100 µL aliquots of the reaction mixture were quenched with 10 µL of 10% (v/v) formic acid and kept at room temperature before HPLC analysis. With these conditions, the CoA production increased linearly with time until 50 sec. The HPLC conditions for monitoring the consumption of MLC and production of CoA are listed in **Table 7.**

**Table 7: HPLC conditions for MLC and CoA separation and quantification.**

| | |
|---|---|
| **Column** | ZORBAX Eclipse XDB-C18 250x4.6 mm, Phenomenex^{®} |
| **Solvent A** | H₂O + TFA 0.1% |
| **Solvent B** | Acetonitrile + TFA 0.1% |
| **Flow** | 1 mL/min |
| **Injection volume** | 20 µL |
| **Gradient** | 0-3 min at 5% B |
| | 3-6 min at 6.5% B |
| | 6-10 min at 9 % B |
| | 10-14 min at 90 % B |
| | 14-18 min at 5% |
| **Wavelength** | 260 nm |

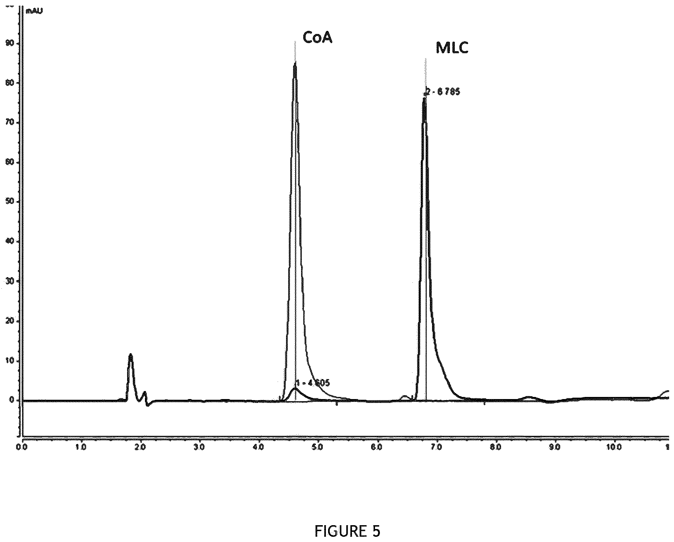

CoA and MLC are well separated with respective retention time of 4.60 min and 6.78 min as shown in **Figure 5****.**

### 1.2.3. Phloroglucinol production assay in E. coli

Pre-cultured *E. coli* BL21(DE3) cells transformed as described in paragraph 1.2.4 above were inoculated into medium M9 containing kanamycin (Glucose 2%; Mineral salts (Na₂HPO₄ 18 g/L, KH₂PO₄ 3.13 g/L, NaCl 0.53 g/L, NH₄Cl 2.11 g/L); MgSO₄ 2mM ; CaCl₂ 0.2 mM ; Trace metals 1x (4.5 mg/L ZnSO₄, 1 mg/L H₃BO₃, 0.3 mg/L CuSO₄, 1 mg/L MnCl₂, 15 mg/L Na₂ EDTA, 0.3 mg/L CoCl, 0.4 mg/L Na₂MoO₄ and FeSO₄); Kanamycine 50 µg/mL) and incubated at 20°C overnight after induction with IPTG at 0.1mM when the OD₆₀₀ was 0.7. The extracellular concentration of Phlo was then measured with colorimetric reagent as described in section 1.2.3 above.

### 2. EXAMPLE 2: CHARACTERIZATION OF THE MUTANT POLYPEPTIDES OBTAINED - RESULTS

### 2.1 Yeast studies

Computational experimental studies carried out according to Example 1 allowed to predict the three-dimensional structure of the functional homodimer of PhlD_Tpu and to uncover molecular determinants involved in substrate and product recognition. Using these original and complementary computer-aided strategies, combined with literature reviews, several amino acid positions were predicted as targets for mutagenesis to improve key properties of the enzyme, including the catalytic activity and stability of the enzyme. A series of yeast mutants were then constructed and screened for improved Phlo production. The best yeast mutants were finally characterized. In this section, we describe the active mutants identified. A summary of the mutants with their measured Phlo production activity in yeast is provided in **Table 9.**

Next, using the best yeast mutant M21 (A124P, A154P, S177A, S236P, A270S, A298D) and a variant missing the mutation A270S (A124P, A154P, S177A, S236P, A298D; named M26) as new templates, the best mutations identified for positions 268 and 271, were introduced to create new yeast mutants that revealed in some cases an increase in Phlo production, up to 86% compared to PhlD_Tpu **(**T**able** 8).

From all the yeast mutants tested, yeast mutant M27 that contains seven mutations (A124P, A154P, S177A, S236P, A298D, E268Q and E271R) was one of the best Phlo producer with a relative activity compared to WT of about 190%. Stability and enzyme kinetics of M27 variant were further characterized *in vitro* and compared to WT.

The stability of M27 was investigated by nano DSF experiments and confirmed the better thermostability of M27 with an improved Tₘ (66.7°C), 5°C higher than the measured value for native sequence **(Fig**u**re 6**).

Evaluation of the reaction kinetics showed that the k_{cat} of yeast M27 is reduced by a factor of 1.3 while the *K*_{M} is twice lower than that of the PhlD_Tpu WT, resulting in a 1.6 times higher catalytic efficiency of this variant compared to PhlD_Tpu WT (**Tables 8 and 9**).

**Table 8: PhlD_Tpu WT and M27 kinetic parameters**

| **Kinetic parameters** | **PhlD_Tpu_M27** | **PhlD_Tpu WT** |
|---|---|---|
| *k*_{cat} (*s*⁻¹) | 0.28 ±0.06 | 0.38 |
| *K*_{M} (µM) | 4.9 ± 0.544 | 10.52 |
| *k*_{cat} / *K*_{M} (s⁻¹ . µM⁻¹) | 0.057 | 0.036 |

**Table 9: List of designed Phld_Tpu yeast mutants from libraries 1-12, with similar (% Phlo production ≥90% relative to WT) or higher phloroglucinol production activity relative to Phld_Tpu WT. For each yeast mutant, positions, activity as well as protein scaffold in which mutations were incorporated are indicated. Relative activity refers to the ratio of the amount of phloroglucinol in gram per litter (G/L) obtained in the supernatant after 3 days of culture of the mutant, to the amount of phloroglucinol in gram per litter (G/L) obtained in the supernatant after 3 days of culture of the WT in the same conditions.**

| Mutant | Library | Mutations | Relative activity vs WT (% Phlo production) | Phlo prod. (mg/L) | ID | Scaffold |
|---|---|---|---|---|---|---|
| 1 | Library 12 | A124P, A154P, S177A, S236P, E268Q, E271R, A298D | 186 | 112,9 | M27 | M26 |
| 2 | Library 12 | A124P, A154P, S177A, S236P, E268T, E271K, A298D | 175 | 104,2 | M28 | M26 |
| 3 | Library 12 | A124P, A154P, S177A, S236P, E268S, A270S, E271K, A298D | 170 | 90,6 | -- | M21 |
| 4 | Library 12 | A124P, A154P, S177A, S236P, E268Q, E271K, A298D | 170 | 97,4 | -- | M26 |
| 5 | Library 12 | A124P, A154P, S177A, S236P, E268S, E271R, A298D | 170 | 101,3 | -- | M26 |
| 6 | Library 12 | E268Q, E271K | 160 | 71,4 | -- | WT |
| 7 | Library 12 | E268T, E271K | 160 | 114,8 | -- | WT |
| 8 | Library 12 | E268T, E271R | 160 | 93,7 | -- | WT |
| 9 | Library 12 | A124P, A154P, S177A, S236P, E268Q, A270S, E271K, A298D | 160 | 85,1 | -- | M21 |
| 10 | Library 12 | A124P, A154P, S177A, S236P, E268T, A270S, E271R, A298D | 160 | 84,6 | -- | M21 |
| 11 | Library 12 | A124P, A154P, S177A, S236P, E268T, A270S, E271V, A298D | 160 | 70,8 | -- | M21 |
| 12 | Library 12 | A124P, A154P, S177A, S236P, E268S, E271K, A298D | 160 | 96,2 | -- | M26 |
| 13 | Library 5 | Q129M, V1761 | 150 | 78,0 | | WT |
| 14 | Library 12 | E268Q, E271R | 150 | 69,8 | M29 | WT |
| 15 | Library 12 | A124P, A154P, S177A, S236P, E268Q, A270S, E271R, A298D | 140 | 76,9 | -- | M21 |
| 16 | Library 12 | A124P, A154P, S177A, S236P, E268S, A270S, E271R, A298D | 140 | 63,3 | -- | M21 |
| 17 | Library 1 | E271K | 178 | 148,9 | | WT |
| 18 | Library 2 | S35G, A60R, A91R, A123S, A124P, A154P, S177A, S236P, A270S, A298D | 130 | 52,6 | M22 | WT |
| 19 | Library 12 | E268S, E271K | 130 | 69,1 | -- | WT |
| 20 | Library 6 | P93C, A262C | 126 | 65,1 | | WT |
| 21 | Library 12 | E268T, E271V | 125 | 70,8 | -- | WT |
| 22 | Library 12 | A124P, A154P, S177A, S236P, E268T, A270S, E271K, A298D | 125 | 90,6 | -- | M21 |
| 23 | Library 1 | E271R | 171 | 141,8 | | WT |
| 24 | Library 2 | S35G, S46A, A60R, A91R, A123S, A124P, A154P, S177A, M191V, S236P, Q238K, A270S, D290A, A298D | 120 | 66,8 | M23 | WT |
| 25 | Library 3 | R49A | 120 | 54,3 | | WT |
| 26 | Library 4 | M3391 | 120 | 67,1 | | WT |
| 27 | Library 1 | E271V | 114 | 68,1 | | WT |
| 28 | Library 1 | E271M | 110 | 66,0 | | WT |
| 29 | Library 2 | A124P, A154P, S177A, S236P, A270S, A298D | 110 | 50,8 | M21 | WT |
| 30 | Library 4 | M339C | 110 | 58,8 | | WT |
| 31 | Library 12 | A124P, A154P, S177A, S236P, E268Q, A270S, A298D | 110 | 56,4 | -- | M21 |
| 32 | Library 12 | A124P, A154P, S177A, S236P, E268T, E271R, A298D | 110 | 67,8 | | M26 |
| 33 | Library 12 | A124P, A154P, S177A, S236P, E268T, E271V, A298D | 110 | 66,7 | -- | M26 |
| 34 | Library 3 | R49S | 105 | 74,6 | | WT |
| 35 | Library 1 | E268Q | 101 | 56,5 | | WT |
| 36 | Library 3 | D209A | 100 | 44,62 | | WT |
| 37 | Library 5 | V161M | 100 | 55,0 | | WT |
| 38 | Library 1 | E313A | 98 | 66,7 | | WT |
| 39 | Library 1 | E323A | 95 | 51,8 | | WT |
| 40 | Library 3 | V2021 | 95 | 54,8 | | WT |
| 41 | Library 1 | V211I | 94 | 73,0 | | WT |
| 42 | Library 5 | V176L | 94 | 48,8 | | WT |
| 43 | Library 1 | 1212C | 90 | 54,5 | | WT |
| 44 | Library 1 | R58A | 90 | 60,7 | | WT |
| 45 | Library 1 | E61A | 100 | 66,4 | | WT |
| 46 | Library 1 | R64A | 90 | 62,3 | | WT |
| 47 | Library 1 | V66A | 90 | 60,7 | | WT |
| 48 | Library 1 | L139A | 90 | 58,4 | | WT |
| 49 | Library 1 | P207A | 90 | 61,8 | | WT |
| 50 | Library 1 | D206A | 91 | 62,7 | | WT |
| 51 | Library 1 | S214A | 95 | 65,4 | | WT |
| 52 | Library 1 | D254A | 102 | 69,3 | | WT |
| 53 | Library 6 | A262C | 98 | 82,7 | | WT |
| 54 | Library 7 | S35G, T38A, T73A, A123D, A124P, T125A, I127V, Q184H, S236A, Q238R, D290A, D324E, R352L | 100 | 44,1 | | WT |
| 55 | Library 7 | S236A, Q238R, R352L | 100 | 53,2 | | WT |

### 2.2 E. coli studies

The Phlo production activity of *E. coli* BL21 (DE3) cells transformed with a plasmid expressing Phld_Tpu mutants E271K or M27 was compared to that of *E. coli* BL21 (DE3) cells transformed with a plasmid expressing Phld_Tpu WT or with an empty plasmid.

The results of 2 separate experiments comparing E271K to Phld_Tpu WT are presented in Table 10 below and show that *E. coli* BL21 (DE3) E271K mutant shows significantly higher Phlo production activity than *E. coli* BL21 (DE3) Phld_Tpu WT.

**Table 10: Phlo production activity of various E. coli BL21 (DE3) transformed cells.**

| *E. coli* BL21 (DE3) transformed cell | Phlo production in mg/L Experiment 1 | Phlo production in mg/L Experiment 2 |
|---|---|---|
| Phld_Tpu WT | 156.58 | 146.01 |
| E271K | 263.82 | 233.27 |
| Empty plasmid | -0.66 | 3.14 |
| % increase E271K/WT | + 68.5% | +59.8% |

In a further experiment, the Phlo production activity of *E. coli* BL21(DE3) M27 (A124P, A154P, S177A, S236P, E268Q, E271R, and A298D) was compared to *E. coli* BL21 (DE3) Phld_Tpu WT and to *E. coli* BL21 (DE3) transformed with a plasmid expressing *Pseudomonas fluorescens* PhlD_PF. It was here also confirmed that M27 mutant has significantly higher Phlo production activity than Phld_Tpu WT, also in *E. coli* (see **Table 11** below).

**Table 11: Phlo production activity of various E. coli BL21(DE3) transformed cells.**

| *E. coli* BL21 (DE3) transformed cell | Phlo production in mg/L |
|---|---|
| Phld_PF | 67.04 |
| Phld_Tpu WT | 121.77 |
| PhlD_Tpu M27 | 188.39 |
| % increase Phld_Tpu M27/WT | + 54.7% |

### BIBLIOGRAPHIC REFERENCES

Achkar J et al., (2005) "Biosynthesis of phloroglucinol" J Am Chem Soc. 127:5332-5333.
Foley, G.; Mora, A.; Ross, C. M.; Bottoms, S.; Sützl, L.; Lamprecht, M. L.; Zaugg, J.; Essebier, A.; Balderson, B.; Newell, R.; Thomson, R. E. S.; Kobe, B.; Barnard, R. T.; Guddat, L.; Schenk, G.; Carsten, J.; Gumulya, Y.; Rost, B.; Haltrich, D.; Sieber, V.; Gillam, E. M. J.; Bodén, M. Engineering Indel and Substitution Variants of Diverse and Ancient Enzymes Using Graphical Representation of Ancestral Sequence Predictions (GRASP). PLOS Comput. Biol. 2022, 18 (10), e1010633.
Gietz RD. Yeast transformation by the LiAc/SS carrier DNA/PEG method. Methods Mol Biol. 2014;1163:33-44.
Goldenzweig, A.; Goldsmith, M.; Hill, S. E.; Gertman, O.; Laurino, P.; Ashani, Y.; Dym, O.; Unger, T.; Albeck, S.; Prilusky, J.; Lieberman, R. L.; Aharoni, A.; Silman, I.; Sussman, J. L.; Tawfik, D. S.; Fleishman, S. J. Automated Structure- and Sequence-Based Design of Proteins for High Bacterial Expression and Stability. Mol. Cell 2016, 63 (2), 337-346.
Katoh, K.; Misawa, K.; Kuma, K.; Miyata, T. MAFFT: A Novel Method for Rapid Multiple Sequence Alignment Based on Fast Fourier Transform. Nucleic Acids Res. 2002, 30 (14), 3059-3066.
Kimple ME, Brill AL, Pasker RL. Overview of affinity tags for protein purification. Curr Protoc Protein Sci. 2013 Sep 24;73:9.9.1-9.9.23.
Lange, O. F.; Grubmüller, H. Generalized Correlation for Biomolecular Dynamics. Proteins Struct. Funct. Bioinforma. 2006, 62 (4), 1053-1061.
Miller, B. R. I.; McGee, T. D. Jr.; Swails, J. M.; Homeyer, N.; Gohlke, H.; Roitberg, A. E. MMPBSA.Py: An Efficient Program for End-State Free Energy Calculations. J. Chem. Theory Comput. 2012, 8 (9), 3314-3321.
Minh, B. Q.; Schmidt, H. A.; Chernomor, O.; Schrempf, D.; Woodhams, M. D.; von Haeseler, A.; Lanfear, R. IQ-TREE 2: New Models and Efficient Methods for Phylogenetic Inference in the Genomic Era. Mol. Biol. Evol. 2020, 37 (5), 1530-1534.
Needleman et Wunsch. J.Mol. Biol. 48,443-453, 1970
Uhlmann, E. & Peyman, A. (1990) Chemical Reviews, 90, 543-584
WO 2013/045510
WO2019/002798
WO2019/002799
Zallot, R.; Oberg, N.; Gerlt, J. A. The EFI Web Resource for Genomic Enzymology Tools: Leveraging Protein, Genome, and Metagenome Databases to Discover Novel Enzymes and Metabolic Pathways. Biochemistry 2019, 58 (41), 4169-4182.
Zha W, Rubin-Pitel SB and Zhao H. (2006) "Characterization of the substrate specificity of PHLD, a type III polyketide synthase from Pseudomonas fluorescens." J Biol Chem. 281 :32036-32047.

## Claims

1. A mutant polypeptide comprising an amino acid sequence of a phloroglucinol synthase having at least 80 % of identity, preferably at least 85 % of identity, more preferably at least 90 % of identity, with the amino acid sequence of the *Tsukamurella pulmonis* phloroglucinol synthase as set forth in SEQ ID NO: 1, with one or more substitution(s) of one or more amino acid residue(s) selected from the group consisting of:
a) any amino acid residue or combination of amino acid residues located from position 268 to position 271 of SEQ ID NO:1; or
any amino acid residue or combination of amino acid residues located from a position equivalent to position 268 to a position equivalent to position 271 of SEQ ID NO: 1, after optimal global alignment with SEQ ID NO:1; or
b) an amino acid residue located at position 49 of SEQ ID NO:1; or
an amino acid residue located at a position equivalent to position 49 of SEQ ID NO:1, after optimal global alignment with SEQ ID NO:1; or
c) an amino acid residue located at position 339 of SEQ ID NO:1; or
an amino acid residue located at a position equivalent to position 339 of SEQ ID NO:1, after optimal global alignment with SEQ ID NO:1; or
d) amino acid residues located at positions 93 and 262 of SEQ ID NO:1; or
amino acid residues located at a positions equivalent to positions 93 and 262 of SEQ ID NO:1, after optimal global alignment with SEQ ID NO:1; or
e) amino acid residues located at positions 129 and 176 of SEQ ID NO:1; or
amino acid residues located at positions equivalent to positions 129 and 176 of SEQ ID NO:1, after optimal global alignment with SEQ ID NO:1; and
f) any combination thereof.

2. The mutant polypeptide according to claim 1, comprising one or more substitutions of any amino acid residue or combination of amino acid residues located from position 268 to position 271 of SEQ ID NO:1, or comprising one or more substitutions of any amino acid residue or combination of amino acid residues located from a position equivalent to position 268 to a position equivalent to position 271 of SEQ ID NO: 1, after optimal global alignment with SEQ ID NO:1.

3. The mutant polypeptide according to claim 2, comprising:
a) a substitution of the glutamic acid (E) residue located at position 271 of SEQ ID NO:1, or of the amino acid residue located at a position equivalent to position 271 of SEQ ID NO: 1 after optimal global alignment with SEQ ID NO:1, preferably wherein the glutamic acid residue located at position 271 of SEQ ID NO:1, or the amino acid residue located at a position equivalent to position 271 of SEQ ID NO: 1 after optimal global alignment with SEQ ID NO:1, is substituted by an amino acid residue selected from the group consisting of arginine (R), histidine (H), lysine (K), glutamine (Q), valine (V), methionine (M), phenylalanine (F), leucine (L), tyrosine (Y), and proline (P); preferably substituted by an amino acid residue selected from the group consisting of arginine (R), histidine (H), lysine (K), valine (V), methionine (M), and phenylalanine (F) ; more preferably substituted by an amino acid residue selected from the group consisting of arginine (R), lysine (K), valine (V), and methionine (M); even more preferably substituted by an amino acid residue selected from the group consisting of arginine (R), lysine (K), and valine (V); even more preferably substituted by an amino acid residue selected from the group consisting of arginine (R), and lysine (K);
b) substitutions of the glutamic acid (E) residues located at positions 268 and 271 of SEQ ID NO:1, or of the amino acid residues located at positions equivalent to positions 268 and 271 of SEQ ID NO: 1 after optimal global alignment with SEQ ID NO:1, preferably wherein:
- the glutamic acid (E) residue located at position 268 of SEQ ID NO:1, or the amino acid residue located at a position equivalent to position 268 of SEQ ID NO: 1 after optimal global alignment with SEQ ID NO:1, is substituted by an amino acid residue selected from Q, T, and S; and
- the glutamic acid (E) residue located at position 271 of SEQ ID NO:1, or the amino acid residue located at a position equivalent to position 271 of SEQ ID NO: 1 after optimal global alignment with SEQ ID NO:1, is substituted by an amino acid residue selected from K, R and V; or
c) substitutions of amino acid residues located at positions 124, 154, 177, 236, 268, 271 and 298 of SEQ ID NO:1 or of amino acid residues located at positions equivalent to positions 124, 154, 177, 236, 268, 271 and 298 of SEQ ID NO:1, preferably wherein:
- the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
- the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
- the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A); and
- the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
- the glutamic acid (E) residue located at position 268 of SEQ ID NO:1, or the amino acid residue located at a position equivalent to position 268 of SEQ ID NO: 1 after optimal global alignment with SEQ ID NO:1, is substituted by an amino acid residue selected from Q, T, and S; and
- the glutamic acid (E) residue located at position 271 of SEQ ID NO:1, or the amino acid residue located at a position equivalent to position 271 of SEQ ID NO: 1 after optimal global alignment with SEQ ID NO:1, is substituted by an amino acid residue selected from K, R and V; and
- the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue; or
d) substitutions of amino acid residues located at positions 124, 154, 177, 236, 268, 270, 271 and 298 of SEQ ID NO:1 or of amino acid residues located at positions equivalent to positions 124, 154, 177, 236, 268, 270, 271 and 298 of SEQ ID NO:1, preferably wherein:
- the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
- the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
- the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A); and
- the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
- the glutamic acid (E) residue located at position 268 of SEQ ID NO:1, or the amino acid residue located at a position equivalent to position 268 of SEQ ID NO: 1 after optimal global alignment with SEQ ID NO:1, is substituted by an amino acid residue selected from Q, T, and S; and
- the alanine (A) residue located at position 270 of SEQ ID NO:1, or the amino acid residue located at a position equivalent to position 270 of SEQ ID NO: 1 after optimal global alignment with SEQ ID NO:1, is substituted by a serine (S) residue; and
- the glutamic acid (E) residue located at position 271 of SEQ ID NO:1, or the amino acid residue located at a position equivalent to position 271 of SEQ ID NO: 1 after optimal global alignment with SEQ ID NO:1, is substituted by an amino acid residue selected from K, R and V; and
- the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue; or
e) substitutions of amino acid residues located at positions 35, 60, 91, 123, 124, 154, 177, 236, 270, and 298 of SEQ ID NO:1 or of amino acid residues located at positions equivalent to positions 35, 60, 91, 123, 124, 154, 177, 236, 270, and 298 of SEQ ID NO:1, preferably wherein:
- the serine (S) residue located at position 35 of SEQ ID NO:1, or the amino acid residue located at a position equivalent to position 35 of SEQ ID NO: 1 after optimal global alignment with SEQ ID NO:1, is substituted by a glycine (G) residue; and
- the alanine (A) residue located at position 60 of SEQ ID NO:1, or the amino acid residue located at a position equivalent to position 60 of SEQ ID NO: 1 after optimal global alignment with SEQ ID NO:1, is substituted by an arginine (R) residue; and
- the alanine (A) residue located at position 91 of SEQ ID NO:1, or the amino acid residue located at a position equivalent to position 91 of SEQ ID NO: 1 after optimal global alignment with SEQ ID NO:1, is substituted by an arginine (R) residue; and
- the alanine (A) residue located at position 123 of SEQ ID NO:1, or the amino acid residue located at a position equivalent to position 123 of SEQ ID NO: 1 after optimal global alignment with SEQ ID NO:1, is substituted by a serine (S) residue; and
- the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
- the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
- the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A) residue; and
- the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
- the alanine (A) residue located at position 270 of SEQ ID NO:1, or the amino acid residue located at a position equivalent to position 270 of SEQ ID NO: 1 after optimal global alignment with SEQ ID NO:1, is substituted by a serine (S) residue; and
- the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue; or
f) substitutions of amino acid residues located at positions 35, 46, 60, 91, 123, 124, 154, 177, 191, 236, 238, 270, 290 and 298 of SEQ ID NO:1 or of amino acid residues located at positions equivalent to positions 35, 46, 60, 91, 123, 124, 154, 177, 191, 236, 238, 270, 290 and 298 of SEQ ID NO:1, preferably wherein:
- the serine (S) residue located at position 35 of SEQ ID NO:1, or the amino acid residue located at a position equivalent to position 35 of SEQ ID NO: 1 after optimal global alignment with SEQ ID NO:1, is substituted by a glycine (G) residue; and
- the serine (S) residue located at position 46 of SEQ ID NO:1, or the amino acid residue located at a position equivalent to position 46 of SEQ ID NO: 1 after optimal global alignment with SEQ ID NO:1, is substituted by an alanine (A) residue; and
- the alanine (A) residue located at position 60 of SEQ ID NO:1, or the amino acid residue located at a position equivalent to position 60 of SEQ ID NO: 1 after optimal global alignment with SEQ ID NO:1, is substituted by an arginine (R) residue; and
- the alanine (A) residue located at position 91 of SEQ ID NO:1, or the amino acid residue located at a position equivalent to position 91 of SEQ ID NO: 1 after optimal global alignment with SEQ ID NO:1, is substituted by an arginine (R) residue; and
- the alanine (A) residue located at position 123 of SEQ ID NO:1, or the amino acid residue located at a position equivalent to position 123 of SEQ ID NO: 1 after optimal global alignment with SEQ ID NO:1, is substituted by a serine (S) residue; and
- the alanine (A) residue located at position 124 of SEQ ID NO:1, or the amino acid residue located at a position equivalent to position 124 of SEQ ID NO: 1 after optimal global alignment with SEQ ID NO:1, is substituted by a proline (P) residue; and
- the alanine (A) residue located at position 154 of SEQ ID NO:1, or the amino acid residue located at a position equivalent to position 154 of SEQ ID NO: 1 after optimal global alignment with SEQ ID NO:1, is substituted by a proline (P) residue; and
- the serine (S) residue located at position 177 of SEQ ID NO:1, or the amino acid residue located at a position equivalent to position 177 of SEQ ID NO: 1 after optimal global alignment with SEQ ID NO:1, is substituted by an alanine (A) residue; and
- the methionine (M) residue located at position 191 of SEQ ID NO:1, or the amino acid residue located at a position equivalent to position 191 of SEQ ID NO: 1 after optimal global alignment with SEQ ID NO:1, is substituted by a valine (V) residue; and
- the serine (S) residue located at position 236 of SEQ ID NO:1, or the amino acid residue located at a position equivalent to position 236 of SEQ ID NO: 1 after optimal global alignment with SEQ ID NO:1, is substituted by a proline (P) residue; and
- the glutamine (Q) residue located at position 238 of SEQ ID NO:1, or the amino acid residue located at a position equivalent to position 238 of SEQ ID NO: 1 after optimal global alignment with SEQ ID NO:1, is substituted by a lysine (K) residue; and
- the alanine (A) residue located at position 270 of SEQ ID NO:1, or the amino acid residue located at a position equivalent to position 268 of SEQ ID NO: 1 after optimal global alignment with SEQ ID NO:1, is substituted by a serine (S) residue; and
- the aspartic acid (D) residue located at position 290 of SEQ ID NO:1, or amino acid located at a position equivalent to position 290 of SEQ ID NO: 1 after optimal global alignment with SEQ ID NO:1, is substituted by an alanine (A) residue; and
- the alanine (A) residue located at position 298 of SEQ ID NO:1, or the amino acid residue located at a position equivalent to position 298 of SEQ ID NO: 1 after optimal global alignment with SEQ ID NO:1, is substituted by an aspartic acid (D) residue.

4. The mutant polypeptide according to any of claims 1 to 3, comprising the amino acid sequence as defined in SEQ ID NO:2: 
MNTTEQQSVLPQQAWLGAPPAPPTSVAVIESLATX35SPTQTYGQAEX46ADX49VAARFDDPRQX60ERI
RRVYAKTRVTERHLAIDPLTPEFAEFSX91RX93DTVRERMDLFFEHAAPLAIETARRALGDNX123X124TDI
GX129LVFVTSTGFLAPGVDVAVIRALGLX154PQTSRVVINFMGCAAAMNALRX176X177TDYVRAQPDRK
SLX191ICLELSSVNAVFSGDPNDVVISSLFADGCGAAVIGASEVGHPLPX236GX238IVVRDTFTHLLDGAE
DGIVLGVNX262NGITCX268LX270X271SLPQYIVNGVAPVVDAVLX290RNGLGREX298VAHWAIHPGGPK
IIESAAAALGLPDDASRLSWDVLAEHGNX339LSVSLLFVLERLRAQVASDGADGAPSTGMAFSFAPGVT
VEGFLFDVVTTGE;
 wherein :
a) the amino acid residue X₂₇₁ is any amino acid residue selected from the group consisting of arginine (R), histidine (H), lysine (K), glutamine (Q), valine (V), methionine (M), phenylalanine (F), leucine (L), tyrosine (Y), and proline (P); preferably the amino acid residue X₂₇₁ is selected from the group consisting of arginine (R), histidine (H), lysine (K), valine (V), methionine (M), and phenylalanine (F) ; more preferably the amino acid residue X₂₇₁ is selected from the group consisting of arginine (R), lysine (K), valine (V), and methionine (M), even more preferably the amino acid residue X₂₇₁ is selected from the group consisting of arginine (R), lysine (K), and valine (V);
and
b) the amino acid residues X₃₅, X₄₆, X₄₉, X₆₀, X₉₁, X₉₃, X₁₂₃, X₁₂₄, X₁₂₉, X₁₅₄, X₁₇₆, X₁₇₇, X₁₉₁, X₂₃₆, X₂₃₈, X₂₆₂, X₂₆₈, X₂₇₀, X₂₉₀, X₂₉₈, and X₃₃₉ are independently selected from any amino acid ;
preferably wherein :
i. the amino acid residue X₃₅ is the amino acid residue S or G, preferably G;
ii. the amino acid residue X₄₆ is the amino acid residue S or A, preferably A;
iii. the amino acid residue X₄₉ is any amino acid residue selected from the group consisting of R, A and S, preferably X₄₉ is any amino acid residue A or S;
iv. the amino acid residue X₆₀ is the amino acid residue A or R, preferably R;
v. the amino acid residue X₉₁ is the amino acid residue A or R, preferably R;
vi. the amino acid residue X₉₃ is the amino acid residue P or C, preferably C;
vii. the amino acid residue X₁₂₃ is the amino acid residue A or S, preferably S;
viii. the amino acid residue X₁₂₄ is the amino acid residue A or P, preferably P;
ix. the amino acid residue X₁₂₉ is the amino acid residue Q or M, preferably M;
x. the amino acid residue X₁₅₄ is the amino acid residue A or P, preferably P;
xi. the amino acid residue X₁₇₆ is any amino acid residue selected from the group consisting of V, I and L; preferably X₁₇₆ is L or I, preferably I;
xii. the amino acid residue X₁₇₇ is the amino acid residue S or A, preferably A;
xiii. the amino acid residue X₁₉₁ is the amino acid residue M or V, preferably V;
xiv. the amino acid residue X₂₃₆ is the amino acid residue S or P, preferably P;
xv. the amino acid residue X₂₃₈ is the amino acid residue Q or K, preferably K;
xvi. the amino acid residue X₂₆₂ is the amino acid residue A or C, preferably C;
xvii. the amino acid residue X₂₆₈ is any amino acid residue selected from the group consisting of E, Q, T, S, C, G, K, M, and R; preferably X₂₆₈ is any amino acid residue selected from the group consisting of Q, T, S, C, G, K, M, and R; preferably X₂₆₈ is any amino acid residue selected from the group consisting of Q, T, and S;
xviii. the amino acid residue X₂₇₀ is the amino acid residue A or S, preferably S;
xix. the amino acid residue X₂₉₀ is the amino acid residue D or A, preferably A;
xx. the amino acid residue X₂₉₈ is the amino acid residue A or D, preferably D;
xxi. the amino acid residue X₃₃₉ is any amino acid residue selected from the group consisting of M, I, Q, and T; preferably X₃₃₉ is any amino acid residue selected from the group consisting of I, Q, and T, preferably X₃₃₉ is I; or
xxii. any combination thereof.

5. The mutant polypeptide according to any of the preceding claims, comprising the amino acid sequence set forth in SEQ ID NO: 1, with one substitution or a group of substitutions selected from the group consisting of:
1a. A124P, A154P, S177A, S236P, E268Q, E271R, and A298D (mutant polypeptide M27);
2a. A124P, A154P, S177A, S236P, E268T, E271K, and A298D (mutant polypeptide M28);
3a. A124P, A154P, S177A, S236P, E268S, A270S, E271K, and A298D;
4a. A124P, A154P, S177A, S236P, E268Q, E271K, and A298D;
5a. A124P, A154P, S177A, S236P, E268S, E271R, and A298D;
6a. E268Q and E271K;
7a. E268T and E271K;
8a. E268T and E271R;
9a. A124P, A154P, S177A, S236P, E268Q, A270S, E271K, and A298D;
10a. A124P, A154P, S177A, S236P, E268T, A270S, E271R, and A298D;
11a. A124P, A154P, S177A, S236P, E268T, A270S, E271V, and A298D;
12a. A124P, A154P, S177A, S236P, E268S, E271K, and A298D;
13a. Q129M and V176I;
14a. E268Q and E271R (mutant polypeptide M29);
15a. A124P, A154P, S177A, S236P, E268Q, A270S, E271R, and A298D;
16a. A124P, A154P, S177A, S236P, E268S, A270S, E271R, and A298D;
17a. E271K;
18a. S35G, A60R, A91R, A123S, A124P, A154P, S177A, S236P, A270S, and A298D (mutant polypeptide M22);
19a. E268S and E271K;
20a. P93C and A262C;
21a. E268T and E271V;
22a. A124P, A154P, S177A, S236P, E268T, A270S, E271K, and A298D;
23a. E271R;
24a. S35G, S46A, A60R, A91R, A123S, A124P, A154P, S177A, M191V, S236P, Q238K, A270S, D290A, and A298D (mutant polypeptide M23);
25a. R49A; and
26a. M339I.

6. The mutant polypeptide according to any of the preceding claims, which comprises, or consists essentially of, or consists of, an amino acid sequence selected from the group consisting of SEQ ID NO: 3-28.

7. The mutant polypeptide according to any of the preceding claims, further comprising one or more additional mutation(s), such as mutation(s) selected from the group consisting of substitution, deletion, and insertion; preferably wherein the one or more additional mutation(s), is(are) silent or conservative mutation(s), in particular when the one or more additional mutation(s) is(are) substitution(s).

8. A nucleic acid molecule comprising a nucleic acid sequence encoding the mutant polypeptide according to any one of claims 1 to 7, preferably wherein:
a) the nucleic acid molecule is isolated; or
b) the nucleic acid molecule further comprises a promoter controlling expression of the nucleic acid sequence; or
c) the isolated nucleic acid molecule further comprises a transcription terminator controlling expression of the nucleic acid sequence; or
d) the nucleic acid sequence is further optimized for expression in a host cell, in particular a yeast or a bacterium; or
e) any combination of a) to d)
preferably wherein the nucleic acid molecule comprises, or consists essentially of, or consists of, a nucleic acid sequence having at least 80% identity, more preferably at least 85% identity, even more preferably at least 90% identity, with a nucleic acid sequence selected from the group consisting of SEQ ID NO: 46-72.

9. The nucleic acid molecule according to claim 8, wherein:
a) when the nucleic acid molecule further comprises a promoter controlling the expression of the nucleic acid sequence, the promoter is an exogenous promoter, in particular a yeast promoter; or
b) when the isolated nucleic acid molecule further comprises a transcription terminator controlling the expression of the nucleic acid sequence, the transcription terminator is an exogenous terminator, such as a yeast terminator; or
c) the nucleic acid sequence is further optimized for expression in yeast.

10. A vector comprising at least one nucleic acid molecule according to any one of claims 8 to 9, or expressing the mutant polypeptide according to any one of claims 1 to 7, wherein the vector is preferably a plasmid.

11. A host cell comprising or expressing the mutant polypeptide according to any one of claims 1 to 7, or containing the nucleic acid molecule according to any one of claims 8 or 9, or containing the vector according to claim 10, or any combination thereof.

12. The host cell according to claim 11, which is a yeast or a bacterium; preferably a yeast selected from the group consisting of the genera *Saccharomyces, Candida, Ashbya, Dekkera, Pichia (Hansenula), Debaryomyces, Clavispora, Lodderomyces, Yarrowia, Zigosaccharomyces, Schizosaccharomyces, Torulaspora, Kluyveromyces, Brettanomycces, Cryptococcus* et *Malassezia* ; more preferably a yeast selected from the species *Saccharomyces cerevisiae, Saccharomyces boulardii, Saccharomyces douglasii, Saccharomyces bayanus, ligosaccharomyces bailii, Schizosaccharomyces pombe, Dekkera brucelensis, Dekkera intermedia, Brettanomycces custersii, Brettanomycces intermedius, Kluyveromyces themotolerens, Torulaspora globosa ou Torulaspora glabrata* or a bacterium selected from the group consisting of the genera *Escherichia, Pseudomonas, Streptomyces, Corynebacterium, Bacillus,* and *Lactobacillus ;* even more preferably a yeast of the *Saccharomyces* genus or a bacterium of the *Escherichia* genus, preferably a yeast of the *Saccharomyces cerevisiae* species or a bacterium of the *Escherichia coli* species.

13. A composition comprising the mutant polypeptide according to any one of claims 1 to 7, the nucleic acid molecule according to any one of claims 8 or 9, the vector according to claim 10, the host cell according to claim 11 or 12, or any combination thereof.

14. A use of the mutant polypeptide according to any one of claims 1 to 7, the nucleic acid molecule according to any one of claims 8 or 9, the vector according to claim 10, the host cell according to claim 11 or 12, or any combination thereof, for producing phloroglucinol.

15. A method for producing phloroglucinol, which comprises the steps of:
a) contacting a host cell expressing the mutant polypeptide as defined in any one of claims 1 to 7, with a suitable substrate;
b) in vitro culture of the host cell of step a) under conditions permitting expression of the nucleic acid molecule contained in said host cell, so as to produce phloroglucinol;
c) optionally recovering the phloroglucinol-containing culture medium obtained after step b); and
d) optionally, purifying phloroglucinol from the culture medium of step c).

16. A method for producing phloroglucinol, which comprises the following steps:
a) contacting the mutant polypeptide as defined in any one of claims 1 to 7 with malonyl-CoA ;
b) incubating the mixture from step a) under conditions suitable for producing phloroglucinol;
c) optionally recovering the phloroglucinol-containing reaction medium obtained after step b); and
d) optionally, purifying phloroglucinol from the reaction medium of step c).
